# EUROPEAN PATENT SPECIFICATION

(11) **EP 2 635 288 B1**
(45) Date of publication and mention of the grant of the patent: **29.07.2015**
(21) Application number: 11838813.1
(22) Date of filing: 03.11.2011
(51) Int. Cl.: A61K 31/52, A61K 38/00, A61K 49/00, G01N 33/50, A61P 35/00

(54) **TREATMENT OF PITUITARY CORTICOTROPH TUMORS USING R-ROSCOVITINE**
BEHANDLUNG CORTICOTROPER HYPOPHYSENTUMORE MIT R-ROSCOVITIN
TRAITEMENT DE TUMEURS CORTICOTROPES HYPOPHYSAIRES UTILISANT LA R-ROSCOVITINE

(30) Priority: 06.05.2011 US 201161483223 P; 03.11.2010 US 409756 P
(43) Date of publication of application: 11.09.2013
(73) Proprietor: Cedars-Sinai Medical Center, Los Angeles, CA 90048 (US)
(72) Inventor: MELMED, Shlomo, Los Angeles California 90035 (US); LIU, Ning-Ai, Los Angeles California 90077 (US)
(74) Representative: Banford, Paul Clifford
(86) International application number: PCT/US2011/059151
(87) International publication number: WO 2012/061601

(56) References cited:
- EP-A1- 0 911 634
- US-A1- 2005 222 054
- "Pathway-dependent Tumors in Mice Noninvasive Imaging of Spontaneous Retinoblastoma Updated version", Cancer Res Marc Vooijs Jos Jonkers, 1 January 2002 (2002-01-01), pages 1862-1867, XP055117253, Retrieved from the Internet: URL:http://cancerres.aacrjournals.org/cont ent/62/6/1862.full.pdf [retrieved on 2014-05-09]
- MADALINA MUSAT ET AL: "Cyclins and their related proteins in pituitary tumourigenesis", MOLECULAR AND CELLULAR ENDOCRINOLOGY, vol. 326, no. 1-2, 15 September 2010 (2010-09-15), pages 25-29, XP055117284, ISSN: 0303-7207, DOI: 10.1016/j.mce.2010.03.017
- STEVEN W J LAMBERTS ET AL: "Future treatment strategies of aggressive pituitary tumors", PITUITARY, KLUWER ACADEMIC PUBLISHERS, BO, vol. 12, no. 3, 12 November 2008 (2008-11-12), pages 261-264, XP019733008, ISSN: 1573-7403
- WARBRICK E. ET AL.: 'A small peptide inhibitor of DNA replication defines the site of interaction between the cyclin-dependent kinase inhibitor p21WAF1 and proliferating cell nuclear antigen.' CURRENT BIOLOGY vol. 5, no. 3, 01 March 1995, pages 275 - 282, XP004585355
- LIU N.-A. ET AL.: 'Pituitary Corticotroph Ontogeny and Regulation in Transgenic Zebrafish.' MOLECULAR ENDOCRINOLOGY vol. 17, no. 5, May 2003, pages 959 - 966, XP055084914
- LIU N.-A.ET AL.: 'Targeting zebrafish and murine pituitary corticotroph tumors with a cyclin-dependent kinase (CDK) inhibitor.' PNAS vol. 108, no. 20, 17 May 2011, pages 8414 - 8419, XP055084918
- DE AZEVEDO ET AL.: 'Inhibition of cyclin-dependent kinases by purine analogues Crystal structure of human cdk2 complexed with roscovitine.' EUR. J. BIOCHEM. vol. 243, no. 1-2, 15 January 1997, pages 518 - 526, XP002084931
- HELSETH A. ET AL.: 'Transgenic Mice That Develop Pituitary Tumors: A Model For Cushing's Disease.' AMERICAN JOURNAL OF PATHOLOGY vol. 140, no. 5, May 1992, pages 1071 - 1080, XP055084919
- JORDAN S. ET AL.: 'Cyclin D and cyclin E expression in normal and adenomatous pituitary.' EUROPEAN JOURNAL OF ENDOCRINOLOGY vol. 143, no. 1, July 2000, pages R1 - R6, XP055084923
- FUKUOKA H. ET AL.: 'EGFR as a therapeutic target for human, canine, and mouse ACTH-secreting pituitary adenomas.' JOURNAL OF CLINICAL INVESTIGATION vol. 121, no. 12, 01 December 2011, pages 4712 - 4721, XP055084927
- MEIJER L. ET AL.: 'Biochemical and cellular effects of roscovitine, a potent and selective inhibitor of the cyclin-dependent kinases cdc2, cdk2 and cdk5.' EUR. J. BIOCHEM. vol. 243, no. 1-2, 15 January 1997, pages 527 - 536, XP002922858
- PAPRSKAROVA M. ET AL.: 'Functional p53 in cells contributes to the anticancer effect of the cyclin- dependent kinase inhibitor roscovitine.' JOURNAL OF CELLULAR BIOCHEMISTRY vol. 107, no. 3, 01 June 2009, pages 428 - 437, XP055084928
- ROUSSEL-GERVAIS A. ET AL.: 'Cooperation between Cyclin E and p27Kip1 in Pituitary Tumorigenesis.' MOLECULAR ENDOCRINOLOGY vol. 24, no. 9, September 2010, pages 1835 - 1845, XP055084931
- VESELY J. ET AL.: 'Inhibition of cyclin-dependent kinases by purine analogues.' EUR. J. BIOCHEM. vol. 224, no. 2, 01 September 1994, pages 771 - 786, XP000576956
- VLOTIDES G. ET AL.: 'Pituitary Tumor-Transforming Gene: Physiology and Implications for Tumorigenesis.' ENDOCRINE REVIEWS vol. 28, no. 2, April 2007, pages 165 - 186, XP055084932

## Description

This invention relates to the treatment of pituitary tumors and related disease conditions.

### BACKGROUND

Despite being small (< 2mm) and often undetectable by MRI, pituitary corticotroph tumors are associated with significant morbidities and mortality due to adrenal glucocorticoid (Gc) hypersecretion in response to autonomous tumor ACTH production¹. The standard of care for Cushing's disease consists of transsphenoidal pituitary tumor resection, pituitary-directed radiation, adrenalectomy and/or medical suppression of adrenal gland cortisol production. While transsphenoidal ACTH-secreting tumor resection yields 30-70% surgical cure rate, adenoma recurrence rate is high². Efficacies of other therapeutic modalities are limited by factors such as slow therapeutic response, development of pituitary insufficiency, and uncontrolled pituitary tumor growth in the face of adrenal gland resection or inhibition^{2,3}. Effective pharmacotherapy directly targeting corticotroph tumor growth and/or ACTH production remains a major challenge⁴.

The pituitary is highly sensitive to cell cycle disruptions^{5,6}. Pituitary tumors acquire oncogene and tumor suppressor genetic and epigenetic alterations, which result in unrestrained proliferation, aberrant neuroendocrine regulatory signals and disrupted humoral milieu, mediated directly or indirectly by dysregulated cyclin-dependent kinases (CDKs)^{5,7}. Although CDK gene mutations have not readily been identified in human pituitary tumors, overexpression of cyclins and dysregulation of CDK inhibitors are common features of pituitary adenomas, indicating that CDK activation has important pathological and potential therapeutic implications^{8,9}.

Small molecule CDK inhibitors are being evaluated for cancer therapy, some of which have led to clinical trials for lymphoma, lung and nasopharyngeal cancers^{10,11}. Preclinical studies of CDK inhibitors, however, are often hampered by the requirement for large drug quantity, and prolonged duration of administration to observe potential efficacy. While the genetic spectrum of tumor associated mutations and/or their cellular context may dictate specific CDK dependence, particular CDK inhibitors may not have been tested in the most appropriate tumor types in vivo^{11,12}. Animal models faithfully recapitulating human pituitary tumors would enable rapid and efficient testing to identify small molecule CDK inhibitors with optimal potency.

Regardless of cell lineage origin, pituitary tumors almost invariably overexpress pituitary tumor transforming gene (PTTG), which encodes a securin that binds separase in the APC complex, and governs faithful chromosome segregation during mitosis¹³ PTTG was originally isolated from rat pituitary tumor cells¹⁴. Dysequilibrium of intracellular PTTG abundance leads to cell cycle disruption and neoplastic formation, causing chromosomal instability and aneuploidy, and also aberrant G1/S and G2/M transition by transcriptional dysregulation of cyclin expression^{13, 15-20}. On the other hand, PTTG overexpession also triggers irreversible cell cycle arrest in pituitary growth hormone (GH)- and gonadotropin (LH, FSH)-expressing tumors by activating lineage-specific senescence pathways, contributing to the benign propensity of pituitary tumors^{13,21} .

Musat et al. (2010) Molecular and Cellular Endocrinology Vol 326 (No.1-2) p25-29 provides a summary of the role cylcins (and their related proteins)in pituitary tumourigenesis whereas Lamberts et a/. (2008) Pituitary Vol 12 (no. 3) p261-264 discloses treatment strategies for aggressive pituitary tumours.

There remains a need in the art for alternative and/or improved methods of treating pituitary tumors and particularly, pituitary corticotroph tumors.

### SUMMARY OF THE INVENTION

Various embodiments of the present invention provide oleomoucine and R-roscovitine or salts thereof for use in a method of treating a pituitary corticotroph tumor, suppressing ACTH and/or corticosterone levels in a ACTH-secreting pituitary adenoma, inhibiting the growth of an ACTH-secreting pituitary adenoma, and/or treating Cushing's disease. The method can comprise: providing a composition comprising a selective CDK/cyclin inhibitor; and administering a therapeutically effective amount of the composition to a mammalian subject in need of treating for a pituitary corticotroph tumor, suppressing ACTH and/or corticosterone levels in a ACTH-secreting pituitary adenoma, inhibiting the growth of an ACTH-secreting pituitary adenoma, or treating Cushing_{'}s disease to treat the pituitary corticotroph tumor, suppress the ACTH and/or corticosterone levels in a ACTH-secreting pituitary adenoma, inhibit the growth of an ACTH-secreting pituitary adenoma, or treat Cushing's disease.

In various embodiments, the mammalian subject can be in need of treating a pituitary corticotroph tumor and the pituitary corticotroph tumor is treated. In certain embodiments, the corticotroph tumor is a PTTG overexpressing corticotroph tumor. In other embodiments, the mammalian subject can be in need of suppressing ACTH and/or corticosterone levels in a ACTH-secreting pituitary adenoma and the ACTH and/or corticosterone levels in the ACTH-secreting pituitary adenoma are suppressed. In still other embodiments, the mammalian subject can be in need of inhibiting the growth of an ACTH-secreting pituitary adenoma and the growth of an ACTH-secreting pituitary adenoma is inhibited. In various embodiments, the mammalian subject can be in need of treating Cushing's disease and Cushing's disease is treated.

Oleomoucine and R-roscovitine are selective CDK/cyclin inhibitors and 2,6,9-substituted purine analogues. In various preferred embodiments, the selective CDK/cyclin inhibitor is R-roscovitine or salts thereof.

Other features and advantages of the invention will become apparent from the following detailed description, taken in conjunction with the accompanying drawings, which illustrate, by way of example, various features of embodiments of the invention.

### BRIEF DESCRIPTION OF THE FIGURES

Exemplary embodiments are illustrated in referenced figures. It is intended that the embodiments and figures disclosed herein are to be considered illustrative rather than restrictive.

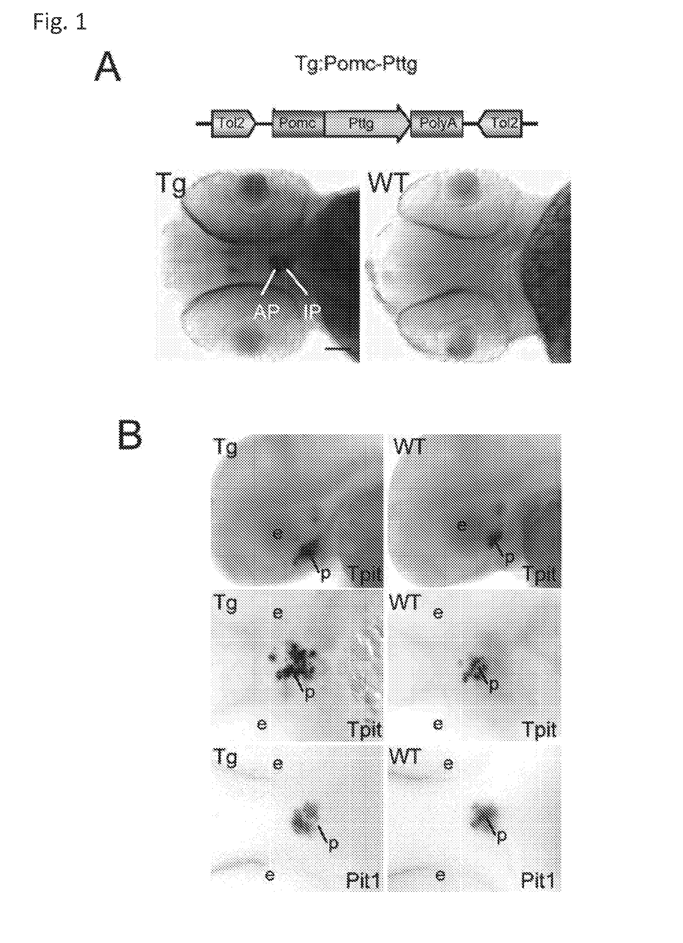

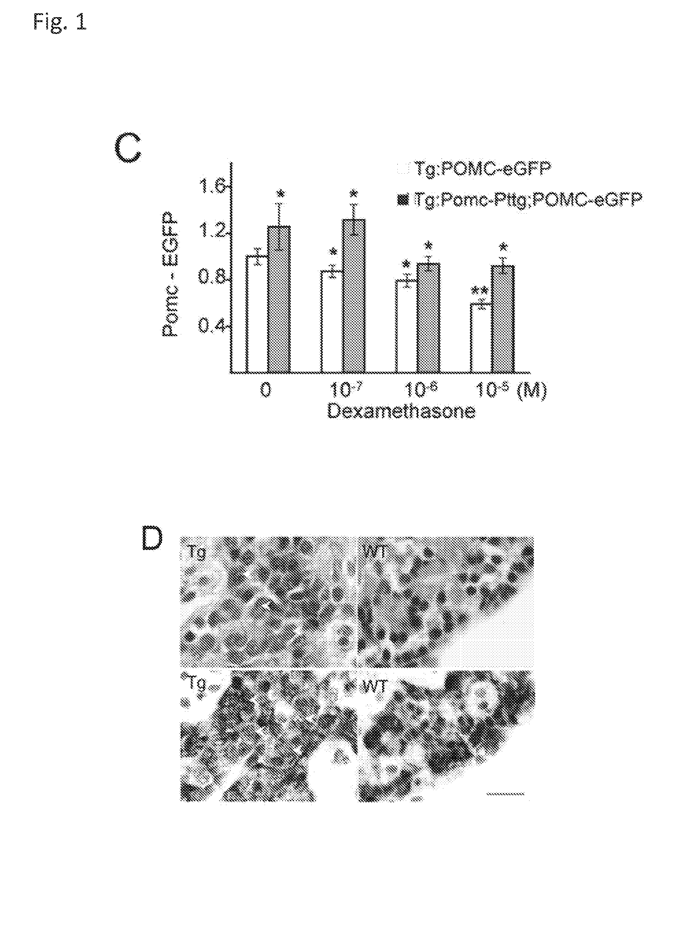

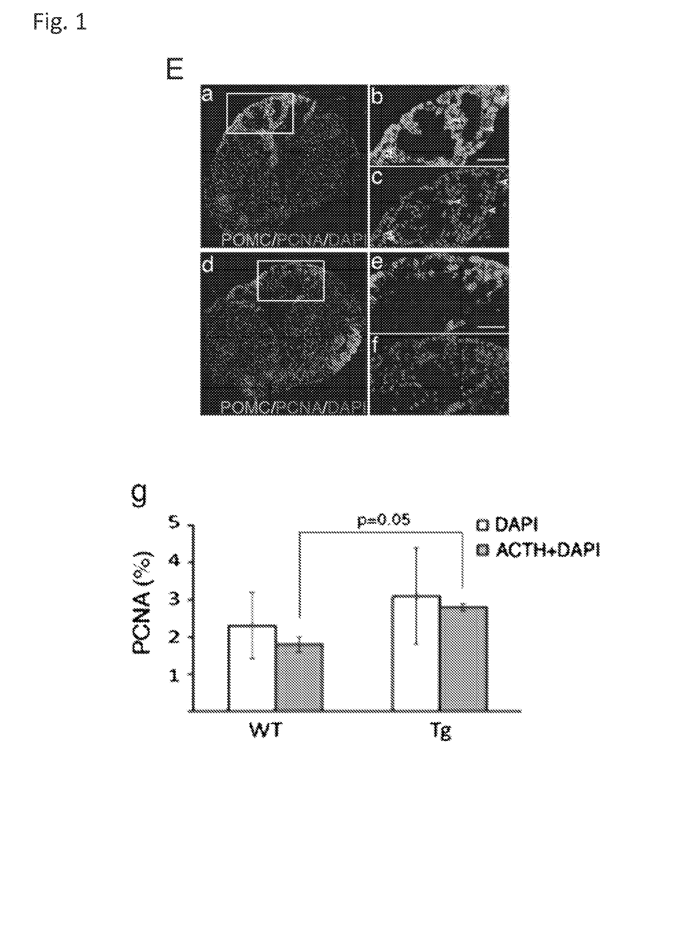

Figure 1 depicts pituitary pathology of zPttg transgenic zebrafish, Tg:Pomc-zPttg in accordance with various embodiments of the present invention. (A) Generation of Tg:Pomc-zPttg fish. Top, schematic representation of Pomc-zPttg transgene. Bottom, pituitary expression of zPttg in Tg:Pomc-zPttg zebrafish at 72 hours post fertilization (hpf). F1 Tg:Pomc-zPttg transgenics were crossed with wild-type zebrafish, resulting in F2 embryos with 50% of the progeny positive (left panel, Tg), and 50% negative (right panel, WT) for pituitary zPttg expression assessed by whole mount in situ analysis. Ventral view, anterior to the left. (B) Tg:Pomc-Pttg embryos showed increased *Tpit*/*Tbx19* expression, while no significant change of *Pit-1* expression by whole mount in situ analysis at 48 hpf. Antisense mRNA probes are indicated at right lower corner of each panel. Top panels, lateral view; middle and bottom panels, ventral view, anterior to the left. (C) Tg:Pomc-Pttg; POMC-eGFP embryos exhibited increased pituitary eGFP signal, and are more resistant to glucocorticoid negative feedback than Tg:Pomc-Pttg-negative siblings. Double transgenic embryos (Tg:Pomc-Pttg; POMC-eGFP) were generated by breeding Tg:Pomc-Pttg fish with an established transgenic line POMC eGFP, where eGFP expression is driven by the same *zPomc* promoter. Fluorescence intensity of POMC-GFP positive cells was measured in live embryos after dexamethasone treatment at 4 dpf. (D) Pituitary hematoxylin/eosin stain (top panels) and ACTH immunohistochemistry (bottom panels) of sections derived from WT, and Tg:Pomc-Pttg transgenic fish (Tg) at 20 months of age. Arrowheads indicate neoplastic mitotic ACTH-expressing cells. (E) Tg:pomc-pttg pituitary exhibited increased number of PCNA and ACTH co-expressing cells. Representative confocal pituitary images of fluorescence immunohistochemistry detecting PCNA (red) and ACTH (green) expression in Tg:Pomc-Pttg (a-c) and WT (d-f). Paraffin slides were counterstained with DAPI (blue). In b and c, arrowheads indicate ACTH-producing cells co-expressing intra-nuclear PCNA. AP, anterior pituitary; IP, pars intermedia. P, pituitary; Scale bar, 50 µm. (g) (mean ± SE, n = 500 cells counted per pituitary, two pituitaries per group; *P = 0.05). AP, anterior pituitary; IP, pars intermedia. P, pituitary. (Scale bar, 50 µm.)

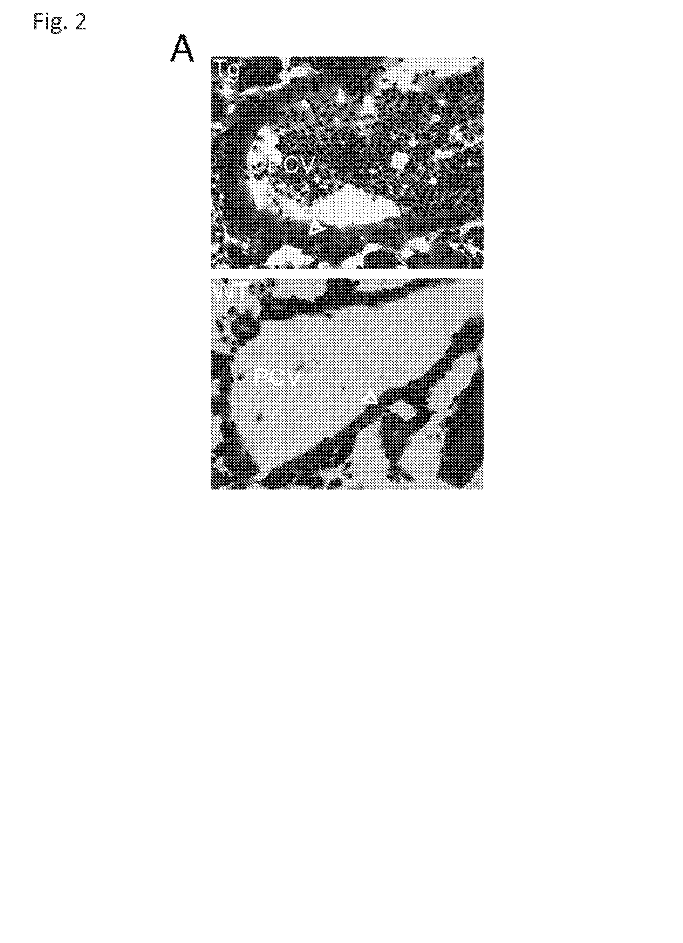

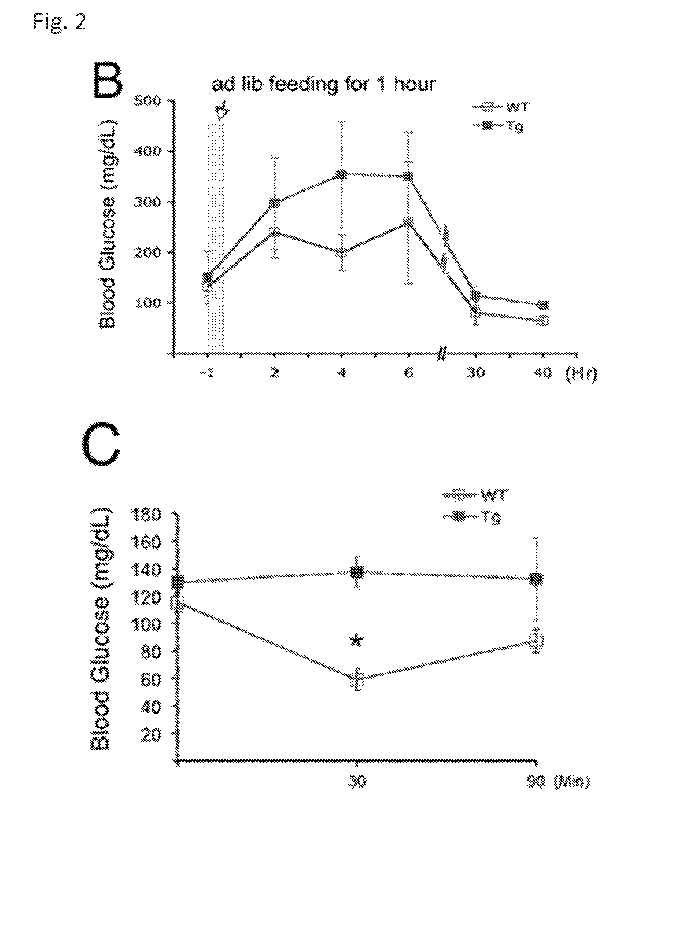

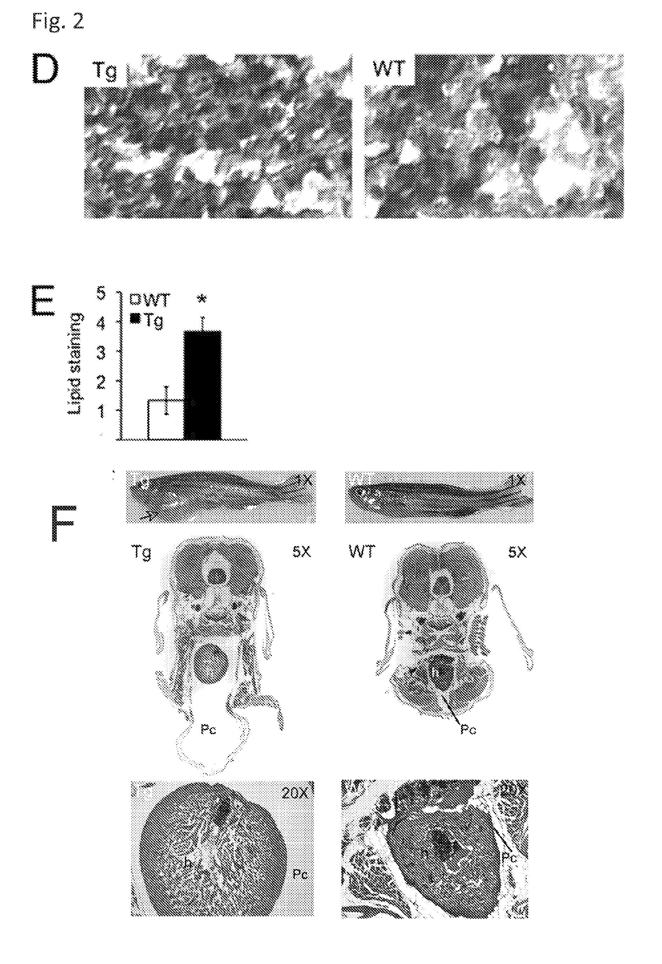

Figure 2 shows that Tg:Pomc-Pttg transgenic zebrafish develop hypercortisolism, exacerbated insulin resistance, glucose intolerance, hepatic steatosis and cardiomyopathy in accordance with various embodiments of the present invention. (A) Tg:pomc-pttg (top panel, Tg) fish showed steroidogenic cell expansion as depicted by hematoxylin/eosin stain of kidney head paraffin slides. Zebrafish glucocorticoid steroidogenic cells are arranged as epithelial cell layers (arrowheads) in association with the renal head posterior cardinal vein (PCV) 28. Tg:pomc-pttg fish also showed blood cell accumulation within the PCV, which was not observed in WT (bottom panel, WT). (B) Tg:pomc-pttg fish showed increased levels of fasting and post-prandial blood glucose levels. Glucose tolerance tests were performed in 72 Tg:pomc-pttg and WT fish. Zebrafish was given ad lib feeding of regular diet for one hour (grey column) after 16 hours of fasting. Blood glucose was measured at different time points after re-feeding. Glucose levels are presented as mean ± SE (AUC, p < 0.0001). (C) Insulin tolerance tests in Tg:pomc-pttg and WT fish. After 20 hours of fasting, zebrafish were intraperitoneally injected with insulin (0.1 unit/100 mg), and blood glucose levels were measured at 30 and 60 min post insulin injection (n = 24, mean ± SE, *, p<0.01). (D) Oil red O (ORO) staining of liver sections reveal hepatic lipid accumulation in Tg:Pomc-pttg transgenics. (E) Area distribution and intensity of hepatic ORO staining were scored (mean ± SE, *, p<0.01). (F) Tg:Pomc-Pttg zebrafish exhibited marked pericardial effusion and ventricular hypertrophy. Top, Tg:Pomc-pttg zebrafish (Tg) with gross pericardial fluid accumulation (arrow) compared with wildtype (WT) at 24 months. Middle, hematoxylin/eosin stain of mid-body cross-section; bottom, high magnification (20x) showing ventricular hypertrophy of the heart. Tg, Tg:Pomc-Pttg; WT, wild-type. Pc, pericardial space; h, heart. Scale bar, 50 µM.

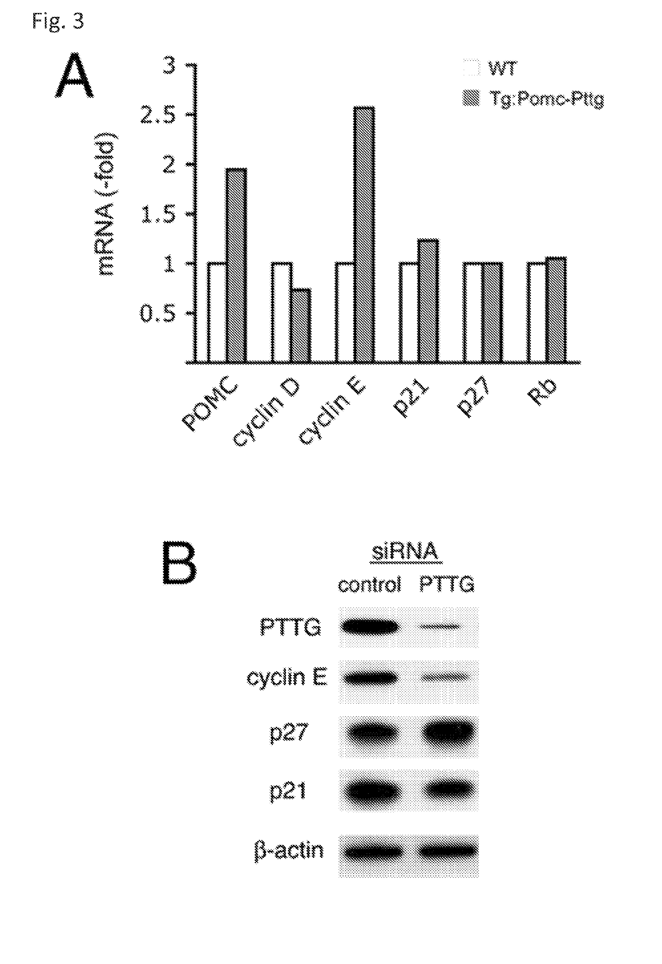

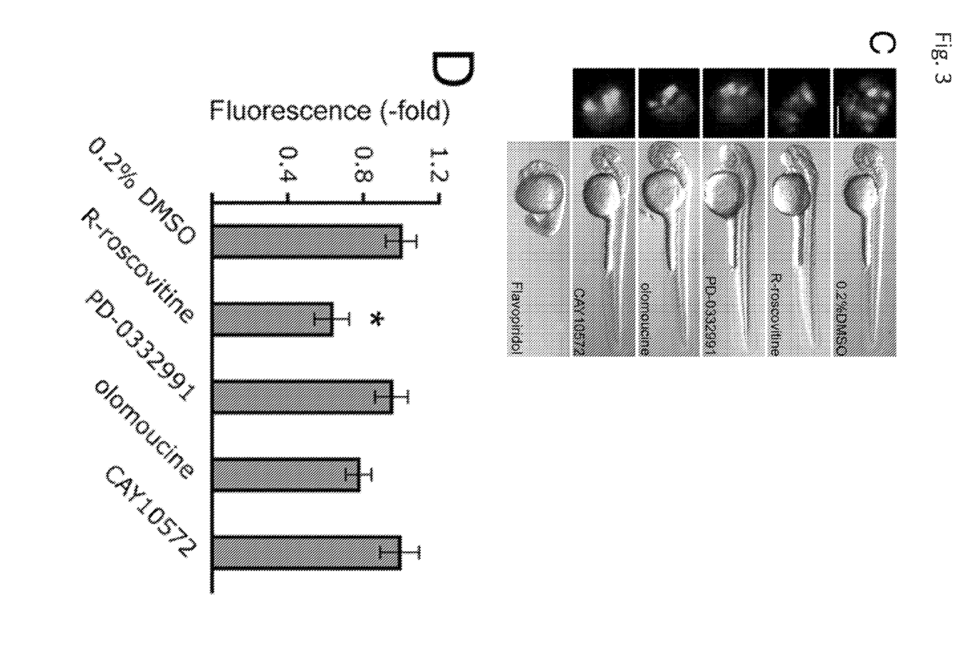

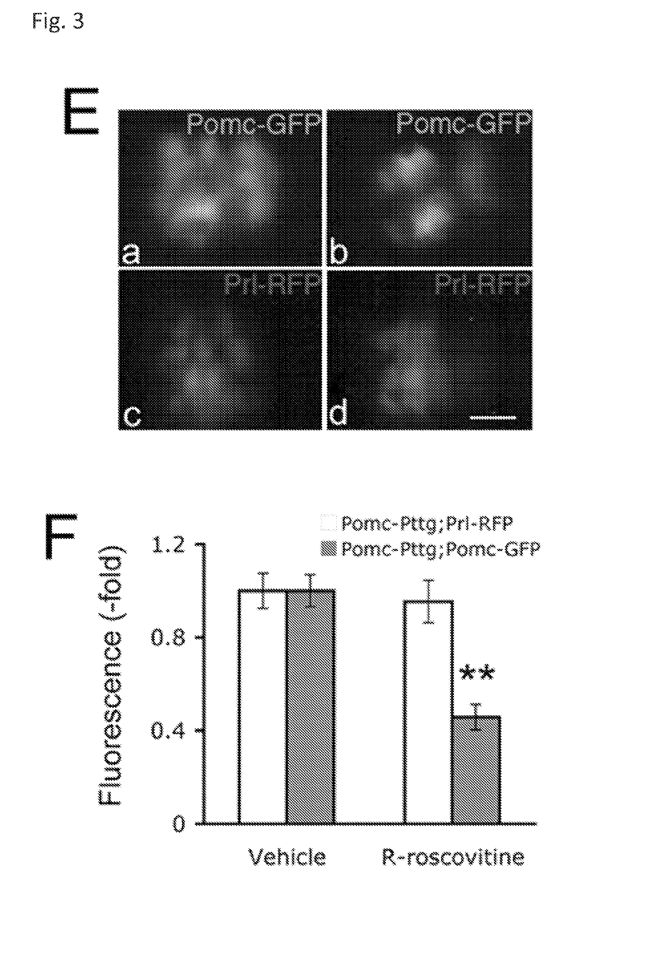

Figure 3 depicts in vivo drug testing in Tg:Pomc-Pttg zebrafish in accordance with various embodiments of the present invention. (A) Pttg overexpression directed by zebrafish *Pomc* promoter induced cyclin E up-regulation in Tg:Pomc-Pttg transgenic pituitary at 3 months. mRNA levels were assayed by quantitative real-time PCR and corrected to β*-actin* (mean ± SE of relative expression. n = 30 pituitaries for each group). (B) Western-blot analysis of mouse corticotroph tumor AtT20 cells transfected with a control or PTTG siRNA. (C) In vivo treatment of Tg:Pomc-Pttg;Pomc-eGFP embryos with different small molecule CDK inhibitors (50 µM) or 0.2% DMSO as control from 18-40 hpf. 100-150 embryos were treated with each compound at a time. Representative images of live embryos are shown with gross morphology (right panels) and pituitary Pomc-GFP positive cells at higher magnification (left panels) at 40 hpf. Embryos exposed to flavopiridol developed early developmental defect before pituitary POMC-cell ontogeny occurs. (D) Relative expression of pituitary Pomc-eGFP fluorescence were analyzed using Velocity 5.2 (Improvision) (mean ± SE of relative expression, n = 7 randomly picked embryos from 100-150 embryos of each group). (E) R-roscovitine specifically suppresses expansion of pituitary POMC cells overexpressing zPttg from 18-48 hpf. Double transgenic Tg:Pomc-Pttg;Prl-RFP embryos were generated by breeding Tg:Pomc-Pttg fish with a previously generated PRL-RFP transgenic line, in which RFP was targeted to pituitary lactotrophs by a zebrafish *Prolactin* promoter³⁵. Representative fluorescent microscopy images of pituitary POMC-eGFP (a and b) and PRL-RFP (c and d) expression in live Tg:Pomc-Pttg;Pomc-eGFP and Tg:Pomc-Pttg;Prl-RFP embryos treated with 0.2% DMSO (a and c) or 50 µM R-roscovitine (b and d). (F) Relative expression of pituitary POMC-eGFP or PRLRFP fluorescence were analyzed using Velocity 5.2 (Improvision) (mean ± SE of relative expression, n = 10 randomly picked embryos from 100-150 embryos of each group). Results represent one of three similar experiments. Scale bar, 50 µm. *, *p<*0.02; **, *p<*0.000005.

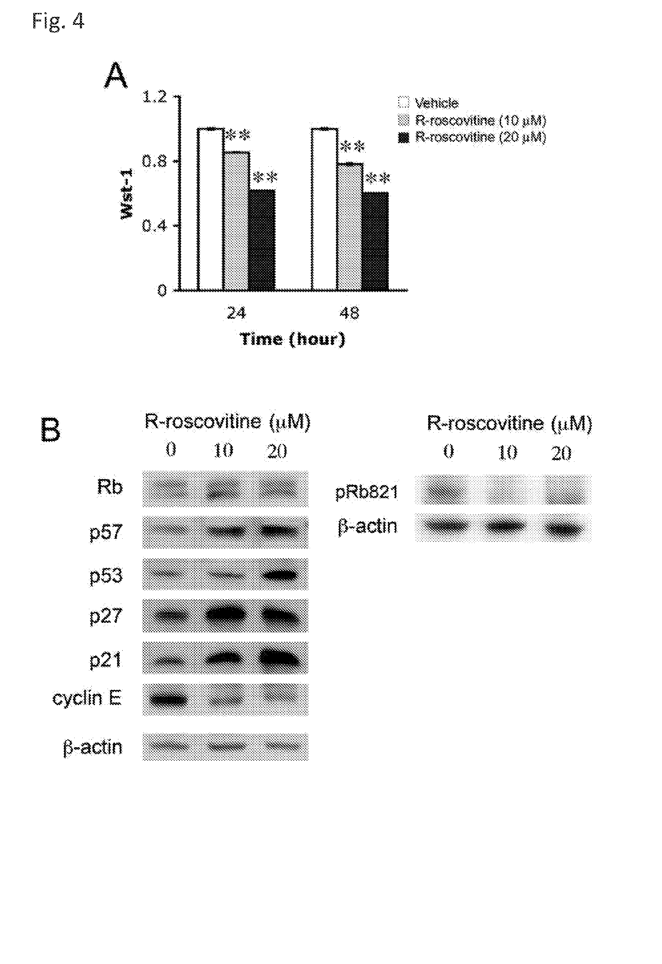

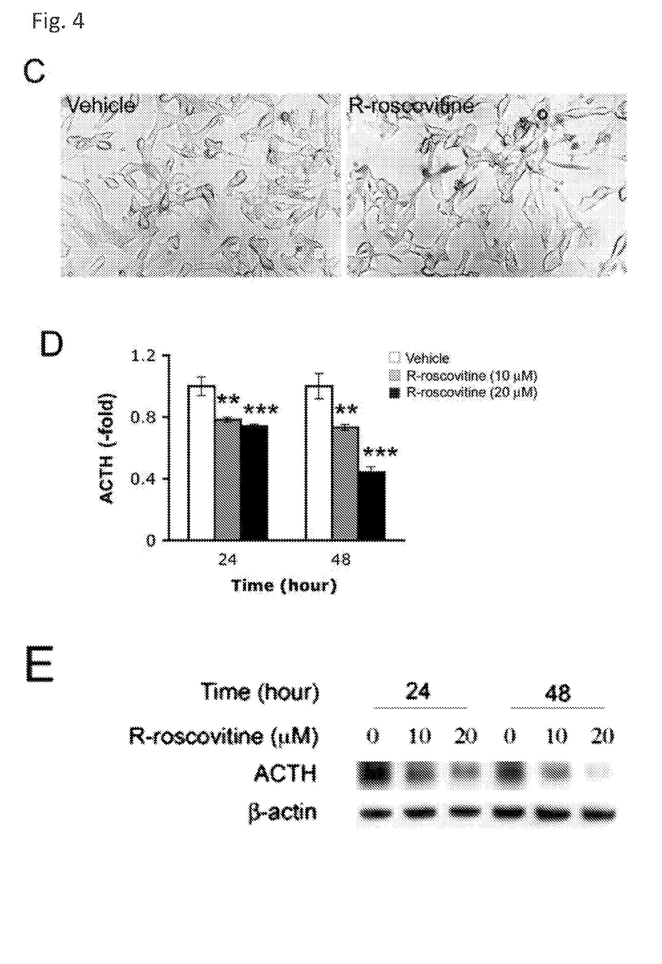

Figure 4 depicts in vitro inhibition of mouse corticotroph tumor cells by R-roscovitine in accordance with various embodiments of the present invention. (A) Treatment of mouse pituitary ACTH-secreting tumor AtT20 cell line with R-roscovitine (1-2 x 10⁻⁵ M) led to decreased number of viable cells at 24 and 48 hours, as depicted by Wst-1 cell proliferation assay (mean ± SE, **, p <0.01). (B) Western-blot analysis of protein extracts derived from AtT20 cells treated with vehicle or R-roscovitine. (C) R-roscovitine treatment (10 µM) of AtT20 cells for 48 hours induced senescence as indicated by increased β-galactosidase expression. (D) ACTH concentration by radioimmuno-assays (RIA) of culture medium from AtT20 cells treated with vehicle or R-roscovitine (mean ± SE, **, p <0.01, ***, p <0.001,). (E) Western-blot analysis of protein extracts derived from AtT20 cells treated with or without R-roscovitine. R-roscovitine inhibits ACTH protein expression in AtT20 cells. Vehicle, 0.2% DMSO.

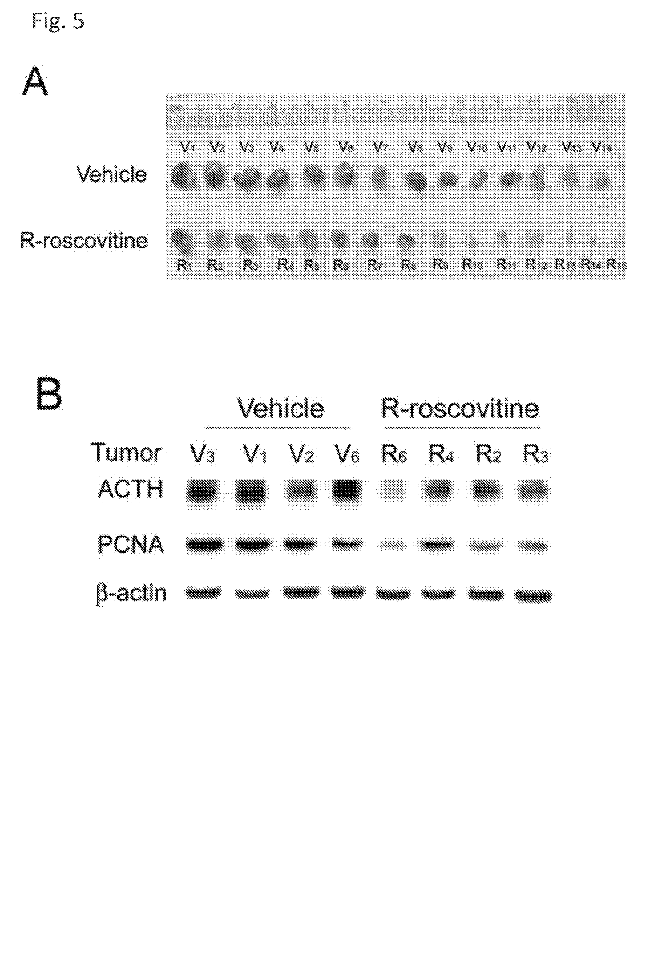

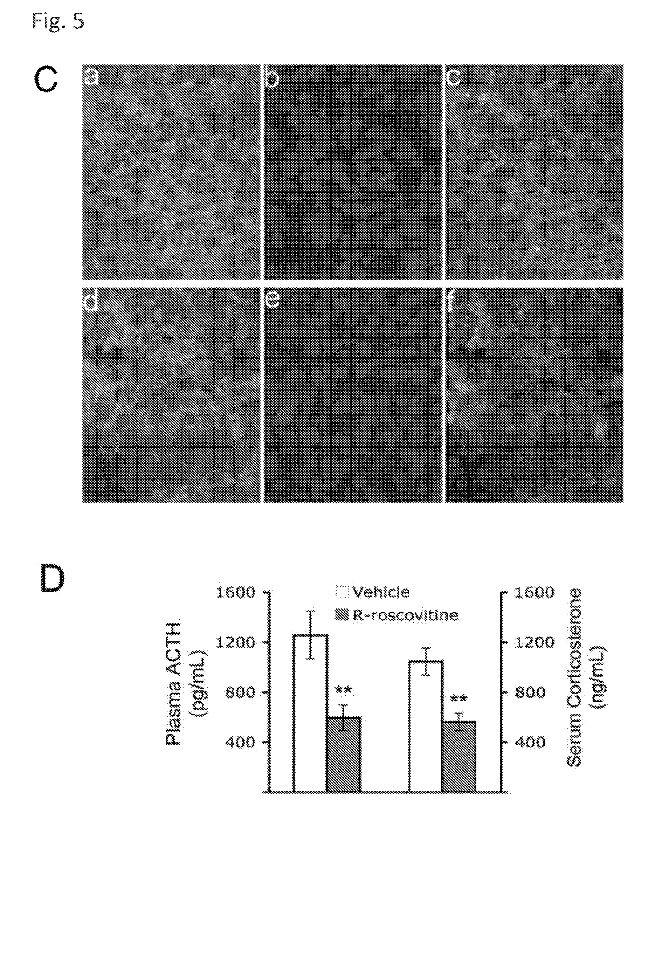

Figure 5 depicts in vivo action of R-roscovitine in mouse corticotroph adenomas in accordance with various embodiments of the present invention. Athymic nude mice were subcutaneously inoculated with corticotroph tumor AtT20 cells (1x10⁵ cells). Three days after tumor cell injection, mice were randomized to receive either R-roscovitine (150 mg/kg) or vehicle by oral gavage twice daily, 5 days per week. After 3 weeks of treatment, AtT20 tumor xenografts were dissected from each animal and (A) tumor volumes were decreased in R-roscovitine-treated animals. (B) Western-blot analyses of representative tumor specimens, which showed decreased ACTH and PCNA protein expression in R-roscovitine treated tumors compared with controls. (C) R-roscovitine treated corticotroph tumors exhibited decreased numbers of PCNA and ACTH co-expressing cells. Representative fluorescence microscopy image of immunohistochemistry detecting PCNA (red) and ACTH (green) expression in control (a-c) and R-roscovitine treated tumors (d-f). Cryosection slides were counterstained with DAPI (blue). (D) Blood was collected from each animal for measurement of plasma ACTH and serum corticosterone levels. R-roscovitine treatment was associated with reduction of blood ACTH and corticosterone levels (mean ± S.E., n = 13-14 mice for each group, **, p < 0.01).

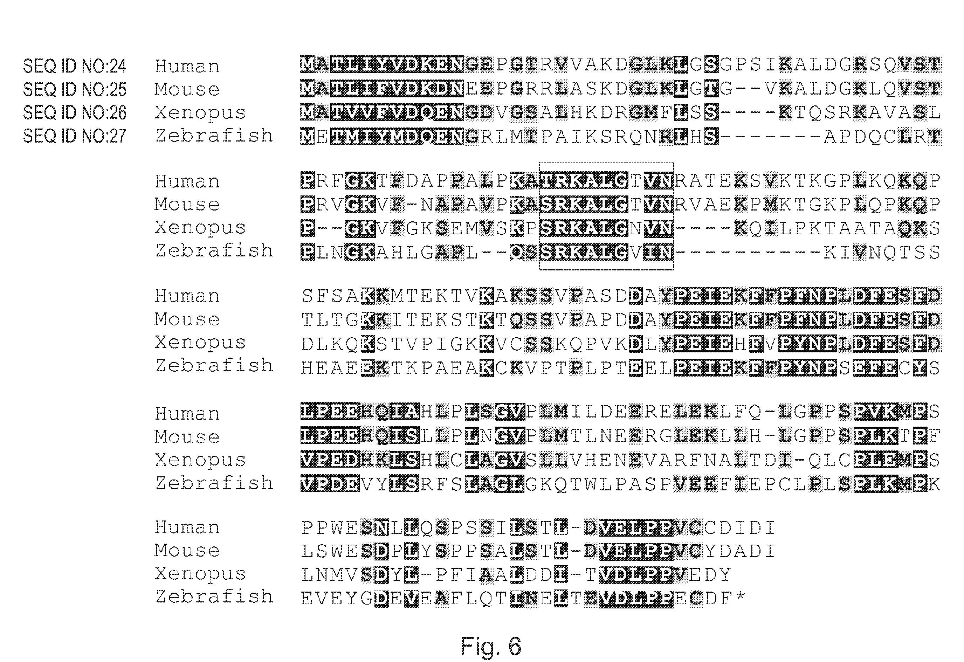

Figure 6 depicts the amino acid sequence alignment of human, mouse, Xenopus, and zebrafish PTTG. Mining the zebrafish genome assembly database (http://www.ensembl.org/Danio_rerio; T. J. P. Hubbard et al., Ensembl 2007 Nucleic Acids Res. 2007 Vol. 35, Database issue: D610-D617) revealed a cDNA sequence encoding a hypothetical 182-aa protein (GenBank accession no. XM_689974). The putative polypeptide sequence shares 76% aa homology with human PTTG (GenBank accession no. NM_004219), with a conserved N-terminal D-box and C-terminal hydrophobic proline rich region. Identical residues are shaded in black, similar residues in gray. The conserved D box is boxed.

### DESCRIPTION OF THE INVENTION

Unless defined otherwise, technical and scientific terms used herein have the same meaning as commonly understood by one of ordinary skill in the art to which this invention belongs. Singleton et al., Dictionary of Microbiology and Molecular Biology 3rd ed., J. Wiley & Sons (New York, NY 2001); March, Advanced Organic Chemistry Reactions, Mechanisms and Structure 5th ed., J. Wiley & Sons (New York, NY 2001); and Sambrook and Russel, Molecular Cloning: A Laboratory Manual 3rd ed., Cold Spring Harbor Laboratory Press (Cold Spring Harbor, NY 2001), provide one skilled in the art with a general guide to many of the terms used in the present application.

One skilled in the art will recognize many methods and materials similar or equivalent to those described herein, which could be used in the practice of the present invention. Indeed, the present invention is in no way limited to the methods and materials described.

"Pituitary tumor" as used herein includes, but is not limited to, lactotrophic adenoma or prolactinoma, ACTH-secreting adenoma, somatotrophic adenoms, corticotrophic adenoma, gonadotrophic adenoma, thyrotrophic adenoms, and null cell adenoma.

"Mammal" as used herein refers to any member of the class *Mammalia,* including, without limitation, humans and nonhuman primates such as chimpanzees and other apes and monkey species; farm animals such as cattle, sheep, pigs, goats and horses; domestic mammals such as dogs and cats; laboratory animals including rodents such as mice, rats and guinea pigs, and the like. The term does not denote a particular age or sex. Thus, adult and newborn subjects, as well as fetuses, whether male or female, are intended to be included within the scope of this term.

"Therapeutically effective amount" as used herein refers to that amount which is capable of achieving beneficial results in a mammalian subject with a pituitary tumor, a pituitary corticotroph tumor, an ACTH-secreting pituitary adenoma and/or or a mammalian subject with Cushing's disease. A therapeutically effective amount can be determined on an individual basis and will be based, at least in part, on consideration of the physiological characteristics of the mammal, the type of delivery system or therapeutic technique used and the time of administration relative to the progression of the disease.

Described herein, the inventors report the generation of a stable transgenic zebrafish with zPttg overexpression targeted to pituitary proopiomelanocortin (POMC) lineages (corticotrophs and melanotrophs). Tg:Pomc-Pttg larvae develop early pathologies reflective of corticotroph tumors including neoplastic corticotrophs with partial Gc resistance, and hypercortisolemia-induced metabolic disturbances in adult transgenic fish. Taking advantage of the early-observed corticotroph pathology, combined with pituitary POMC lineage-specific expression of a fluorescent reporter in live transparent larvae, small molecule CDK inhibitors were tested, which lead to identification of R-roscovitine against PTTG overexpressing corticotrophs. Inhibitory effects of R-roscovitine on corticotroph tumor cells were subsequently validated in an in vivo and in vitro mouse model, supporting use of selective CDK inhibitors as effective therapy for Cushing's disease.

The average diameter of human pituitary corticotroph tumors is 6 mm³⁷, and despite the use of petrosal sinus sampling to establish pituitary ACTH hypersecretion, up to 40% of corticotroph tumors are not visible on MRI, posing significant challenges for surgical resection ³⁸. On the other hand, less commonly encountered large corticotroph tumors may impinge upon surrounding critical structures, thus hampering complete tumor resection. Furthermore, extensive surgical resection may cause significant damage to normal pituitary tissue leading to hypopituitarism in most of these patients. The diagnostic and therapeutic dilemma posed by Cushing's disease is further complicated by significant metabolic and cardiovascular morbidities, and mortality associated with uncontrolled chronic hypercortisolism¹.

Tumor-targeted drug development for Cushing's disease is a major challenge as the pathogenesis of corticotroph adenomas remains enigmatic. Pharmacological protein kinase inhibitors capable of controlling cell growth and metabolism, blocking cell-cycle progression, modulating transcription and inducing apoptosis in cancer cells have been developed for mechanism-based and non-genotoxic target tumor therapies^{10,11}. Recently, protein kinases, e.g., epidermal growth factor receptor (HER) family and cyclin-dependent kinases have been suggested as therapeutic targets for pituitary tumors ^{8, 39, 40}. Although tumor responses to protein kinase inhibitors are selective, and may be dictated by specific mutations and/or tumor cellular context, preclinical testing is hampered by poor predictabilities with respect to molecular pathophysiology of the tumors being assessed. Animal models that faithfully reflect molecular pathogenesis of human disease and allow rapid, non-invasive read-out of tumor-cellular inhibition would facilitate drug testing against corticotroph tumors.

Here, the inventors report generation of germline transgenic zebrafish overexpressing zPttg targeted to pituitary POMC cells, as a small vertebrate animal model of Cushing's disease. While the phenotype of hypercortisolism was observed in adult Tg:Pomc-Pttg zebrafish by 3 months of age, pituitary corticotroph expansion with partial resistance to glucocorticoid negative feedback was already detected within the first 2 days of embryonic development of stable transgenic zebrafish. Furthermore, the Tg:Pomc-Pttg pituitary demonstrates a characteristic feature of human corticotroph adenomas, i.e., cyclin E up-regulation and G1/S phase disruption. The molecular features and early pathologies of corticotroph tumors in Tg:Pomc-Pttg transgenic fish allowed for insight into mechanisms underlying the disease pathogenesis, and also to test drug efficacy in vivo.

Cyclin E overexpression is associated with disrupted G1/S transition contributing to development and progression of breast carcinomas, leukemia and lymphomas³¹. In the pituitary, cyclin E expression is preferentially up-regulated in corticotroph adenomas compared with tumors arising from other lineages, the mechanisms of which remain to be fully defined ^{32, 41, 42}. In a subgroup of corticotroph adenomas, cyclin E up-regulation was associated with loss of Brg1 expression, suggesting the presence of additional cyclin E regulators in corticotrophs²⁷. These results show that corticotroph PTTG overexpression induces cyclin E, while PTTG siRNA suppresses cyclin E expression in murine corticotroph tumor cells (Figure 3). PTTG is overexpressed in more than 90% of pituitary tumors including corticotroph adenomas¹³. In addition to inducing aberrant G1/S and G2/M transition via transcriptional dysregulation of cyclin expression ^{13,15-18}, causing chromosomal instability and aneuploidy, pituitary PTTG overexpression activates lineage-specific senescence pathways triggering irreversible cell cycle arrest in growth hormone (GH)- and gonadotropin (LH, FSH)-expressing tumors^{13, 20, 21}. Corticotroph cyclin E up-regulation may represent another pathway for PTTG-induced pituitary lineage-specific effects, although it is yet unclear whether PTTG regulates cyclin E expression directly or indirectly.

Corticotroph cyclin E up-regulation contributes to cell cycle reentry of differentiated corticotrophs and centrosome instability⁹. To investigate the clinical significance of cyclin E dysregulation in corticotroph adenomas, in vivo drug testing on Tg:Pomc-Pttg embryos was performed using known small molecule compounds with different spectra of CDK/cyclin inhibitory selectivity. These results indicated inhibition of PTTG-overexpressing corticotrophs by the 2,6,9-substituted purine analogues, olomoucine and R-roscovitine, with the latter demonstrating a higher efficacy in vivo (Figure 3). Corticotroph inhibitory effects of R-roscovitine were further validated in mouse corticotroph tumors (Figure 4 and 5). R-roscovitine arrests G1/S or G2/M phases via CDK1/2 inhibition by competing for ATP binding sites¹², inhibition of RNA polymerase II-dependent transcription, and selective action against CDK2/cyclinE^{43, 44}. The molecule is currently undergoing clinical trials for several malignancies, and the oral dosing route and relatively mild side effects of R-roscovitine make daily long-term treatment of Cushing's disease feasible.

These results suggest that the anti-tumor activity of R-roscovitine in corticotroph adenomas involve CDK2/cyclinE and Rb mediated pathways, independent of p53 (Figure 4). Both in vitro and in vivo results show that R-roscovitine also suppresses ACTH expression/production (Figs. 4 and 5), suggesting other regulatory mechanisms in addition to CDK2/cyclin E-mediated cell growth. One of the possible mechanisms may involve inhibition of CRH receptor signaling pathways in corticotroph tumor cells, as 2,6,9-trisubstituted purine analogues have been developed as CRH receptor antagonists exhibiting potential anxiolytic and antidepressant activity ⁴⁵. Further in vivo screening of small molecule libraries with Tg:Pomc-Pttg transgenic fish may lead to identification of compounds with more potent dual effects targeting both corticotroph tumor growth and ACTH production.

The inventors have shown herein that a particular CDK/cyclin inhibitor can suppress pituitary tumor growth and hormone production (e.g., CDK2/cyclin E inhibitor against corticotroph tumor). While not wishing to be bound by any particular theory, the inventors believe that the suppression is via cell lineage and/or CDK inhibitor-specific mechanisms. Therefore, other types of CDK/cyclin inhibitor can also be capable of inhibiting a different types of pituitary tumors, such as somatotroph tumors. As such, the use of CDK/cyclin inhibitors to treat pituitary tumors is included in the embodiments of the present invention.

According to the invention there is provided a composition comprising oleomoucine and R-roscovitine or salts thereof for use as a medicament for administration, in a therapeutically effective amount, to a mammalian subject in need of treating a pituitary corticotroph tumor, suppressing ACTH and/or corticosterone levels in a ACTH-secreting pituitary adenoma, inhibiting the growth of an ACTH-secreting pituitary adenoma, or treating Cushing's disease to treat the corticotroph pituitary tumor, suppress the ACTH and/or corticosterone levels in a ACTH-secreting pituitary adenoma, inhibit the growth of an ACTH-secreting pituitary adenoma, or treat Cushing's disease.

These methods can comprise: providing a composition comprising a selective CDK/cyclin inhibitor and administering a therapeutically effect amount of the composition to the mammalian subject to treat the pituitary tumor, to suppress ACTH and/or corticosterone levels in an ACTH-secreting pituitary adenoma, to inhibit the growth of an ACTH-secreting pituitary adenoma, or to treat Cushing's disease.

Oleomoucine and R-roscovitine are selective CDK/cyclin inhibitors. Examples of other CDK/cyclin inhibitors are provided in Tables 1-3.

**Table 1. Peptidic CDK/cyclin inhibitors**

| CDK Inhibitors | Amino Acids | Sequence | SEQ ID NO: | Target |
|---|---|---|---|---|
| P21 | 15-40 | Not reported | - | CDK2/cyclin E |
| P21 | 58-77 | Not reported | - | CDK2/cyclin E |
| P21 | 17-33 | ACRRLFGPVDSEQLSRD | 3 | CDK2/cyclin E |
| P21 | 63-77 | AWE RVRGLGLPKLY | 4 | CDK2/cyclin E |
| P21 | 141-160 | KRRQTSMTDFYHSKRRLIFS | 5 | PCNA & CDK4/cyclin D1 |
| P21 | 141-160 | KRRQTSMTDFYHSKRRLIFS | 6 | CDK4/cyclin D1 |
| P21 | 141-160 | KRRQTS ATDFYHSKRRLIFS | 7 | CDK4/cyclin D1 |
| P21 | 139-164 | GRKRRQTSMTDFYHSKRRLIFSK RKP | 8 | CDK2/cylin E |
| P21 | 141-160 | KRRQTSMTDFYHSKRRLIFS | 9 | CDK2/cyclin E & PCNA |
| P21 | 141-160 | KRRATSMTDFYHSKRRLIFS | 10 | CDK2/cyclin E |
| P21 | 141-160 | KRRQTSATDFYHSKRRLIFS | 11 | CDK2/cyclin E |
| P21 | 141-160 | KRRQTSMTDFYHSKRRLIAS | 12 | CDK2/cyclin E |
| P21 | 139-164 | GRKRRQTSMTDFYHSKRRLIFSK RKP | 13 | CDK2/cylin E |
| P21 | 139-164 | GRKRRQTSMTDFYHSKRRLIFSK RKP | 14 | CDK2/cylin E |
| P21 | 139-164 | GRKRRQTSMTDFYHSKRRLIFSK RKP | 15 | CDK2/cylin E |
| P21 | 152-159 | HAKRRLIF | 16 | CDK2/cylin A |
| P16 | 84-103 | DAAREGFLDTLVVHRAGAR | 17 | CDK4 & CDK6 |
| E2F | 87-94 | PVKRRLDL | 18 | Cdk2/cyclin A-E2F |
| Rb | 864-880 | SNPPKPLKKRFDIE | 19 | CDK2/cylin A |
| P27 | | Ala-Ala-Abu*-Arg-Lys-Leu-Phe-Gly** | 20 | CDK2/cylin A |
| Rb2/p130 | 641-673 | Spa 310 | - | CDK2 |
| Cyclin A | 285-306 | TYTKKQVLRMEHLVLKVLTFDL | 21 | CDK2/cylin A |
| Cyclin A | 285-306 | TYTKKQVLRMEHLVLKVLTFDL | 22 | CDK2/cylin A |
| CDK2/cyclin A | | NBI1: RWIMYF-NH₂ | 23 | Cyclin A |

**Table 2. Classes of CDK ATP competitive inhibitors and the most representative compound of each category. * in phase I/II clinical trial**

| **CDK/cyclin inhibitors** | **IUPAC** | **Target** |
|---|---|---|
| **Flavonoid Derivatives** | | |
| Flavopyridol* | 2-(2-chlorophenyl)-5,7-dihydroxy-8-((3S,4S)-3-hydroxy-1-methylpiperidin-4-yl)-4H-chromen-4-one | CDK2-4-6-9 |
| P-276-00* | Not reported | CDK2-1-4 |

| **Purine Derivatives** | | |
|---|---|---|
| (R)-roscovitine* | 6-(benzylamino)-9-isopropyl-9H-purin-2-ylamino)butan-1-ol | CDK1-2-5-7-9 |
| olomoucine | Not reported | |
| NU2058 | Not reported | CDK2-1 |

| **Thiazole Derivatives** | | |
|---|---|---|
| SNS-032* | N-(5 -((5 -tert-butyloxazol-2-yl)methylthio)thiazol-2 yl)piperidine-4-carboxamide | CDK2-7-9 |

| **Diaminopyrimidine Derivatives** | | |
|---|---|---|
| R-547* | Not reported | CDK1/cyclin B |
| | | CDK2/cyclinE |
| | | CDK4/cyclinD 1 |

| **Pyridine Derivative** | | |
|---|---|---|
| PD-0332991 * | 1-(2-(5-(piperazin-1-yl)pyridin-2-ylamino)-8-cyclopentyl-5-methylquinazolin-6-yl)ethanone | CDK4-6 |

| **Pyrazole Derivative** | | |
|---|---|---|
| AT-7519 N* | 4-(2,6-dichlorobenzamido)-N-(piperidin-4-yl)-1H-pyrazole-3-carboxamide | CDK1-2-7-9 |

| **Hydroxystaurosporine** | | |
|---|---|---|
| UCN-01 N | Not reported | CDK2, pRb |
| **Indirubin Derivatives** | 5,5'-substituted-indirubin-3-oxime | CDK1-2 |

| **Indole Derivatives** | | |
|---|---|---|
| Indole-3 carbinol | (1H-indol-3-yl)methanol | cyclin D1, cyclin E, CDK2-4-6, p15, p21, p27 |
| **Paullone Derivatives** | dihydro-indolo-benzazepines | CDKs (non-specific) |

| **Hymenialdisine Derivatives** | | |
|---|---|---|
| Hymenialdisine | Not reported | CDKs, GSK-3, ch1 |

**Table 3. Other commercially available small molecule CDK/cyclin inhibitors**

| **CDK/cyclin inhibitors** | **IUPAC** | **Target** |
|---|---|---|
| SU 9516 | Not reported | CDK2-1-4 |
| BML-259 | Not reported | CDK5-2 |
| Purvalanol A | Not reported | CDK2-5-4 |
| Ryuvidine | Not reported | CDK4 |
| AG-024322 | Not reported | CDK1-2-4 |
| Fascaplysin | Not reported | CDK4 |

In various embodiments, the pituitary tumor is a pituitary corticotroph tumor. In various embodiments, the corticotroph tumor is a PTTG overexpressing corticotroph tumor. In various embodiments, the ACTH-secreting pituitary adenoma is a pituitary corticotroph tumor. In various embodiments, the mammalian subject is a human subject.

Olomoucine, and R-roscovitine may be provided as pharmaceutical compositions including a pharmaceutically acceptable excipient along with a therapeutically effective amount of a olomoucine, R-roscovitine or a salt thereof. "Pharmaceutically acceptable excipient" means an excipient that is useful in preparing a pharmaceutical composition that is generally safe, non-toxic, and desirable, and includes excipients that are acceptable for veterinary use as well as for human pharmaceutical use. Such excipients may be solid, liquid, semisolid, or, in the case of an aerosol composition, gaseous.

Pharmaceutical compositions comprising olomoucine, R-roscovitine or a salt thereof may be formulated for delivery via any route of administration. "Route of administration" may refer to any administration pathway known in the art, including but not limited to aerosol, nasal, oral, transmucosal, transdermal, parenteral, or enteral. "Parenteral" refers to a route of administration that is generally associated with injection, including intraorbital, infusion, intraarterial, intracapsular, intracardiac, intradermal, intramuscular, intraperitoneal, intrapulmonary, intraspinal, intrasternal, intrathecal, intrauterine, intravenous, subarachnoid, subcapsular, subcutaneous, transmucosal, or transtracheal. Via the parenteral route, the compositions may be in the form of solutions or suspensions for infusion or for injection, or as lyophilized powders. Via the parenteral route, the compositions may be in the form of solutions or suspensions for infusion or for injection. Via the enteral route, the pharmaceutical compositions can be in the form of tablets, gel capsules, sugar-coated tablets, syrups, suspensions, solutions, powders, granules, emulsions, microspheres or nanospheres or lipid vesicles or polymer vesicles allowing controlled release.

The pharmaceutical compositions can also contain any pharmaceutically acceptable carrier. "Pharmaceutically acceptable carrier" as used herein refers to a pharmaceutically acceptable material, composition, or vehicle that is involved in carrying or transporting a compound of interest from one tissue, organ, or portion of the body to another tissue, organ, or portion of the body. For example, the carrier may be a liquid or solid filler, diluent, excipient, solvent, or encapsulating material, or a combination thereof. Each component of the carrier must be "pharmaceutically acceptable" in that it must be compatible with the other ingredients of the formulation. It must also be suitable for use in contact with any tissues or organs with which it may come in contact, meaning that it must not carry a risk of toxicity, irritation, allergic response, immunogenicity, or any other complication that excessively outweighs its therapeutic benefits.

The pharmaceutical compositions can also be encapsulated, tableted or prepared in an emulsion or syrup for oral administration. Pharmaceutically acceptable solid or liquid carriers may be added to enhance or stabilize the composition, or to facilitate preparation of the composition. Liquid carriers include syrup, peanut oil, olive oil, glycerin, saline, alcohols and water. Solid carriers include starch, lactose, calcium sulfate, dihydrate, terra alba, magnesium stearate or stearic acid, talc, pectin, acacia, agar or gelatin. The carrier may also include a sustained release material such as glyceryl monostearate or glyceryl distearate, alone or with a wax.

The pharmaceutical preparations are made following the conventional techniques of pharmacy involving milling, mixing, granulation, and compressing, when necessary, for tablet forms; or milling, mixing and filling for hard gelatin capsule forms. When a liquid carrier is used, the preparation will be in the form of a syrup, elixir, emulsion or an aqueous or non-aqueous suspension. Such a liquid formulation may be administered directly p.o. or filled into a soft gelatin capsule.

The pharmaceutical compositions may be delivered in a therapeutically effective amount. The precise therapeutically effective amount is that amount of the composition that will yield the most effective results in terms of efficacy of treatment in a given subject. This amount will vary depending upon a variety of factors, including but not limited to the characteristics of the therapeutic compound (including activity, pharmacokinetics, pharmacodynamics, and bioavailability), the physiological condition of the subject (including age, sex, disease type and stage, general physical condition, responsiveness to a given dosage, and type of medication), the nature of the pharmaceutically acceptable carrier or carriers in the formulation, and the route of administration. One skilled in the clinical and pharmacological arts will be able to determine a therapeutically effective amount through routine experimentation, for instance, by monitoring a subject's response to administration of a compound and adjusting the dosage accordingly. For additional guidance, see Remington: The Science and Practice of Pharmacy (Gennaro ed. 20th edition, Williams & Wilkins PA, USA) (2000).

Typical dosages of olomoucine, R-roscovitine or salt thereof can be in the ranges recommended by the manufacturer where known therapeutic compounds are used, and also as indicated to the skilled artisan by the *in vitro* responses or responses in animal models. Such dosages typically can be reduced by up to about one order of magnitude in concentration or amount without losing the relevant biological activity. Thus, the actual dosage will depend upon the judgment of the physician, the condition of the patient, and the effectiveness of the therapeutic method based, for example, on the *in vitro* responsiveness of the relevant primary cultured cells or histocultured tissue sample, such as biopsied malignant tumors, or the responses observed in the appropriate animal models, as previously described.

### EXAMPLES

The following examples are provided to better illustrate the claimed invention and are not to be interpreted as limiting the scope of the invention. To the extent that specific materials are mentioned, it is merely for purposes of illustration and is not intended to limit the invention. One skilled in the art may develop equivalent means or reactants without the exercise of inventive capacity and without departing from the scope of the invention.

### Example 1

### Stable transgenic PTTG overexpression, targeted to pituitary POMC cells rabidly induces early pathologies of Cushing's disease

As an initial step toward identification of novel targets for Cushing's disease therapy, a zebrafish model of pituitary corticotroph tumors was created. Given the highly conserved zebrafish PTTG protein sequence (Figure 6), and while not wishing to be bound by any particular theory, the inventors believe that zebrafish PTTG exhibits conserved properties involving cell cycle dysregulation in pituitary tumor formation²². To test this, the inventors targeted PTTG overexpression to pituitary POMC lineages under the control of the *zPomc* promoter. One- to two-cell stage embryos were co-injected with transposase mRNA and a Tol2 transposon cassette flanking a *zPomc* proximal promoter fused to a full-length zPttg cDNA. Whole mount *in situ* RNA analysis in F2 generation embryos confirmed zPttg overexpression, which temporally and spatially coincided with pituitary POMC cell ontogeny (Figure 1A and ²³). Three independent transgenic lines were maintained, and stable Mendelian transmission for Pomc-Pttg expression is present for more than five generations.

To investigate the effect of zPttg overexpression on embryonic pituitary POMC lineage development, the inventors analyzed highly conserved pituitary transcription factors as markers for both non-POMC (*Pit-1*) and POMC (*Tpit*/*Tbx*-*19*) pituitary lineages²⁴⁻²⁶. At 2 days post fertilization (dpf), Tg:Pomc-Pttg larva demonstrated increased pituitary *Tpit*/*Tbx-19* expression, but *Pit-1* expression was not altered (Figure 1B), indicating early POMC-lineage-specific expansion. Double transgenic embryos (Tg:Pomc-Pttg; POMC-eGFP) were also generated by breeding Tg:Pomc-Pttg zebrafish into a previously established transgenic line, POMC-GFP, where eGFP expression was targeted to pituitary POMC-cells by the same *zPomc* promoter, thus representing a POMC lineage-specific marker²³. Live double transgenic (Tg:Pomc-Pttg; POMC-eGFP) larvae exhibited POMC lineage expansion as evidenced by increased pituitary eGFP expression (Figure 1C).

Pituitary corticotrophs are a critical component of the hypothalamic-pituitary-adrenal (HPA) axis that mediates the stress response via corticotropin-releasing hormone (CRH)-stimulated and subsequently pituitary ACTH stimulated adrenal gland Gc production. Gcs exert negative feedback on CRH and POMC-derived ACTH expression and secretion to restore HPA homeostasis following stress. In human corticotroph tumors, ACTH hypersecretion is partially resistant to Gc negative feedback regulation, further exacerbating uncontrolled hypercortisolism²⁷. To investigate the integrity of the Gc negative feedback pathway in Tg:Pomc-Pttg corticotrophs, live zebrafish embryos were exposed to dexamethasone containing culture medium starting from 10 hours post fertilization (hpf). Pituitary eGFP expression was suppressed in POMC-GFP larvae exposed to 10⁻⁷ M dexamethasone by 4 days post fertilization (dpf) but not in double transgenic (Tg:Pomc-Pttg; POMC-eGFP) larvae, which only exhibited inhibition of pituitary eGFP expression in response to 10 times higher dexamethasone concentrations (10⁻⁶ M) (Figure 1 C), suggesting decreased Gc sensitivity of Tg:Pomc-Pttg corticotrophs. Thus, Tg:Pomc-Pttg corticotrophs rapidly develop the hallmark pathology of ACTH-dependent Cushing's disease within 4 days of embryonic development, i.e., partial glucocorticoid-resistance.

In adult Tg:Pomc-Pttg fish (20 months of age), immunohistochemistry revealed overt neoplastic-appearing pituitary cells with a high nuclear/cytoplasmic ratio, distinct nucleoli and basophilic cytoplasm that stained strongly for ACTH in two of six Tg:Pomc-Pttg pituitary glands analyzed, morphologically resembling human pituitary ACTH-secreting adenomas, while none of six WT pituitary glands showed a similar phenotype (Figure 1D). WT zebrafish pituitary glands exhibited an overall PCNA index of 2.3 ± 0.9% vs. 3.1 ± 1.3% in Tg:Pomc-Pttg (mean ± SE, *p* = 0.6), whereas ACTH-producing cells in the Tg:Pomc-Pttg pituitary exhibited increased PCNA index compared with WT (2.8 ± 0.1% vs. 1.8 ± 0.2%, mean ± SE., *p* = 0.05) (Figure IE), suggesting altered G1/S in neoplastic corticotrophs as a result of zPttg overexpression.

### Example 2

### Hypercortisolism and metabolic disturbance in Tg: Pomc-Pttg zebrafish,

It was tested whether the observed neoplastic corticotroph cell changes in Tg:Pomc-Pttg zebrafish lead to autonomous ACTH secretion and subsequent hypercortisolism. Because one is technically hampered from measuring plasma ACTH or serum cortisol levels by the very limited amount of blood obtainable from each adult zebrafish (∼5 (µl), the inventors measured total cortisol content in age- and weight-matched Tg:Pomc-Pttg zebrafish and their transgene-negative siblings. At 3 months of age, adult Tg:Pomc-Pttg fish showed 40% increased cortisol content vs WT siblings (1.4 ± 0.2 µg/L/mg vs. 1.0 ± 0.2 µg/L/mg, n=12 for each group, mean ± SE, p<0.01). Histological sections of zebrafish kidney were performed to identify zebrafish glucocorticoid steroidogenic cells²⁸. Tg:Pomc-Pttg fish demonstrated increased intra-renal epithelial cell layers surrounding the posterior cardinal vein compared with WT, consistent with ACTH-stimulated adrenal hyperplasia (Figure 2A).

To determine the metabolic impact of hypercortisolism in Tg:Pomc-Pttg zebrafish, adult Tg:Pomc-Pttg and WT fish were subjected to 16 hour fasting followed by ad libitum feeding of regular diet for one hour. Tg:Pomc-Pttg zebrafish exhibited consistently higher levels of fasting and postprandial blood glucose levels than WT zebrafish (96 ± 9 vs. 65 ± 10 mg/dL, mean ± S.D., p< 0.0001) (Figure 2B), demonstrating both attenuated fasting and post-prandial glucose tolerance. Because teleost fish are glucose intolerant due to blunted peripheral responses to insulin²⁹, and glucocorticoids induce insulin resistance in mammals, insulin sensitivity was assessed by testing blood glucose responses to intraperitoneally administered insulin. While WT fish demonstrated a brisk hypoglycemic response 30 minutes after injection of a relatively high insulin dose (0.1 unit/100 mg) (p<0.01), Tg:Pomc-Pttg fish showed no significant change of blood glucose levels for up to 90 minutes after insulin injection (Figure 2C). Hepatic lipid content as detected by oil-Red-O staining was increased in Tg:Pomc-Pttg fish (Figure 2D and E), suggesting visceral adiposity due to increased insulin resistance. Finally, chronic hypercortisolism exerts specific myocardial effects leading to increased ventricular wall thickness with subsequent systolic and diastolic dysfunction contributing to high risk of heart failure in patients with Cushing's disease^{1, 30}. Reflective of the chronic hypercortisolemic status due to corticotroph PTTG overexpression, a spectrum of cardiac hypertrophy was observed in late stage (24 months) Tg:Pomc-Pttg fish, with increased heart wall thickness involving both trabecular and compact zones of the single ventricular chamber (Figure 2F). Four of 18 Tg:Pomc-Pttg transgenic fish also demonstrated co-existing overt pericardial effusion (Figure 2F, top panels). Taken together, corticotroph targeted PTTG overexpression in Tg:pomc-pttg zebrafish results in ACTH-dependent hypercortisolism and metabolic disruptions mimicking features of mammalian Cushing's disease.

### Example 3

### Corticotroph PTTG overexpression, induces cyclin E

Previous studies indicated that PTTG facilitates G1/S transition by acting coordinately with Sp1 to up-regulate cyclin D expression in human choriocarcinoma cells¹⁷. To understand the mechanism for zebrafish corticotroph PTTG overexpression inducing altered G1/S transition (Figure 1), expression of key G1/S cell cycle regulators was analyzed by real-time PCR in adult Tg:Pomc-Pttg and WT pituitary glands. Whereas expression of pituitary cyclin D, p21 and p27 were not different between WT and Tg:Pomc-Pttg, cyclin E mRNA levels were more than doubled in the Tg:Pomc-Pttg pituitary (Figure 3A). Cyclin E up-regulation has been associated with poor clinical outcomes in human malignancies³¹. In the adult pituitary, cyclin E is undetectable in normal cells while preferentially up-regulated in tumors of corticotroph, but not other, lineage(s)³². In murine pituitary POMC cells, cyclin E overexpression collaborates with p27kip1 null mutation to increase cell proliferation, centrosome instability and tumor formation⁹. Up-regulated cyclin E is also associated with loss of Brg1 observed in ∼1/3 of human corticotroph adenomas⁹. Enhanced pituitary cyclin E mRNA levels observed in Tg:Pomc-Pttg fish may not represent protein expression, however the minute adult zebrafish pituitary size (<1 mm) technically hampered analysis of protein expression by Western blot. It was therefore determined whether PTTG regulates cyclin E expression in mammalian (murine) AtT20 corticotroph tumor cells that express abundant endogenous PTTG and cyclin E proteins. Suppression of endogenous PTTG expression with a PTTG-specific siRNA resulted in decreased cyclin E expression and enhanced p27kip1 levels (Figure 3B), while p21 expression was not changed (Figure 3B). These observations suggest that PTTG up-regulation of cyclin E and down-regulation of p27kip1 in pituitary corticotroph tumor cells occurs independently of p21.

### Example 4

### In vivo testing of CDK/cyclin inhibitors in Tg: Pomc-Pttg zebrafish

Zebrafish pituitary POMC cell differentiation starts at the anterior neural ridge by 20 hpf, and is completed within the mature pituitary by 48 hpf 23. Within the first few days of embryonic development, the transgenic fish shown here recapitulate hallmark features of Cushing's disease, i.e., lineage-specific corticotroph expansion with partial glucocorticoid resistance (Figure 1). The observed G1/S alteration, cyclin E up-regulation and neoplastic corticotroph changes led to the screening of small molecule CDK inhibitors with different spectra of inhibitory selectivity, including flavopiridol (CDK 4/6, 2, 1, 9), R-roscovitine (CDK 2, 1)³³, olomoucine (CDK 2, 1)³³, PD-0332991(CDK 4/6), and CAY10572 (CDK 7)³⁴. Tg:Pomc-Pttg; POMC-eGFP double transgenic embryos were exposed to each compound added to the embryo culture medium. While flavopiridol retarded early embryonic development before corticotroph ontogeny occurred, in vivo treatment of zebrafish embryos with R-roscovitine, olomoucine, PD-0332991, and CAY10572 starting at 18 hpf caused no apparent growth defect by 40 hpf (Figure 3C). Strikingly, R-roscovitine-treated embryos exhibited approximately 40% reduction in pituitary POMC-eGFP expression compared with controls (1.0 ± 0.08 vs. 0.6 ± 0.09, mean ± S.E., n = 7 for each group, p< 0.02) (Figures 3C and D). A modest approximately 20% reduction of POMC-eGFP expression was also observed in the olomoucine-treated group (1.0 ± 0.08 vs. 0.8 ± 0.07, mean ± S.E., n = 7 for each group, p = 0.07), whereas PD-0332991 and CAY10572 caused no significant change in pituitary POMC-eGFP expression compared with controls (Figures 3C and D).

To determine the specificity of R-roscovitine action against zPttg-overexpressing POMC cells, another double transgenic line (Tg:Pomc-Pttg;Prl-RFP) was generated by breeding Tg:Pomc-Pttg fish with a previously generated PRL-RFP transgenic line, in which RFP was targeted to pituitary lactotrophs by a zebrafish *Prolactin* promoter 35. In vivo treatment between 18 to 48 hpf of Tg:Pomc-Pttg;Prl-RFP and Tg:Pomc-Pttg;POMC-eGFP embryos with R-roscovitine revealed no effect on Prl-RFP expression (1.0 ± 0.08 vs. 1.0 ± 0.09, mean ± S.E., n = 9 for each group, p = 0.3), but a greater than 50% reduction of POMC-eGFP expression (1.0 ± 0.07 vs. 0.5 ± 0.05, mean ± S.E., n = 10 for each group, p< 0.000005) compared with control groups (Figures 3E and F).

### Example 5

### R-roscovitine action in mousse corticotroph tumor cells

Olomoucine and roscovitine are structurally related 2,6,9-trisubstituted purines, which cause G1/S or G2/M arrest by competing for ATP binding sites on CDK1 and CDK2. The R-isomer of roscovitine (R-roscovitine, CYC202) is a more potent and selective inhibitor of CDK2/cyclinE, and murine corticotrophs are highly sensitive to disrupted CDK2/cyclin E-mediated cell cycle pathways ⁹. Cyclin E up-regulation leads to cell cycle reentry of differentiated POMC cells and also inactivates p27ₖᵢₚ₁, further enhancing cell cycle progression ⁹. In addition, p27ₖᵢₚ₁ protects differentiated pituitary POMC cells from reentering the cell cycle, whereas p57Kip2 is required for cell cycle exit of pituitary precursor cells³⁶. Given the in vivo potency of R-roscovitine against zebrafish Pttg-overexpressing corticotrophs (Figure 3), the inventors studied its effect on CDK2/cyclin E-mediated cell cycle pathways in mouse ACTH-secreting pituitary tumor cells (Figure 4).

Treatment with R-roscovitine (1-2 x 10⁻⁵ M) led to decreased cell number by 24 hours (Figure. 4A). Western-blot analysis of protein extracts derived from R-roscovitine-treated cells revealed evidence for cell cycle arrest including decreased cyclin E, increased p27ₖᵢₚ₁, p57_{Kip2} and p21_{Cip1} expression as well as reduced Thr821 phosphorylation of Rb (Figure. 4B). R-roscovitine treatment also induced senescent features by 48 hours as evidenced by increased β-galactosidase expression (Figure 4C).

Consistent with decreased cell viability, decreased ACTH concentrations was detected in culture medium derived from R-roscovitine-treated AtT20 cells (Figure 4D). Western-blot analysis of protein extracts derived from R-roscovitine-treated AtT20 cells showed suppressed ACTH expression (Figure 4E). These results indicate that R-roscovitine targets cdk2/cyclin E-mediated cell cycle progression, and also inhibits corticotroph ACTH protein expression.

### Example 6

### R-roscovitine inhibits in vivo corticotroph tumor growth and ACTH expression,

To further establish R-roscovitine action on corticotroph tumors in vivo, athymic nude mice (6 ∼ 8 week-old) were injected subcutaneously with AtT20 corticotroph tumor cells (1 x10⁵ cells). Three days after tumor cell injection, 29 of 30 mice had developed small (∼2-3 mm³) but visible subcutaneous tumors, and were randomized to receive either R-roscovitine (150 mg/kg) or vehicle via oral gavage twice daily for five days each week. After three weeks, R-roscovitine caused ∼50% weight reduction of dissected tumor xenografts (40.0 ± 4.7 mg vs. 21.0 ± 2.6 mg, mean ± S.E., n = 13-14 for each group, p < 0.02) (Figure. 5A).

Consistent with the in vitro observations, Western-blot and immunohistochemistry analysis of tumor specimens showed suppressed ACTH and PCNA protein expression by R-roscovitine (Figure 5B and C). R-roscovitine-treated mice exhibited >50% reduction in plasma ACTH levels (1256 ± 596 pg/ml vs. 596 ± 103 pg/ml, mean ± S.E., n = 13-14 for each group, p < 0.01), and ∼50% reduction in serum corticosterone levels (1046 ± 109 ng/ml vs. 561 ± 72 ng/ml, mean ± S.E., n = 13-14 for each group, p < 0.005. Linear regression between ACTH and corticosterone: r = 0.9425, p < 0.0001)(Figure 5D). ]. The high baseline plasma ACTH levels may represent tumor secretion as well as stress-induced responses during CO₂ euthanasia.

### Example 7

### Generation of Tg: pomc-pttg transgenic zebrafish

The zebrafish Pttg EST sequence was identified by Blast searching the zebrafish genome database from Sanger Institute website (GenBank accession number XM_689974). A pair of primers corresponding to zPttg cDNA 5' and 3' coding sequences, zPttg1: 5'-AACGCTGGAC-CTTAGCGAAGACT-3' (SEQ ID NO:1) and zPttg2: 5'-TACTAGAACAGGTTTCTTTATTTTCTTGCGTG-3' (SEQ ID NO:2), were used for PCR amplification to generate a zPttg cDNA containing a complete coding sequence. A tol2 transposon cassette was used to generate the Tg:pomc-pttg transgene, and transgenic founder fish were generated as previously described²³. Briefly, PCR products of the Tg:pomc-pttg transgene construct (without vector DNA) were purified using a GENECLEAN III kit (Bio 101, Vista, CA) and resuspended in 5 mM Tris, 0.5 mM EDTA, 0.1 M KCl at a final concentration of 100 µg/ml. Fertilized embryos from wild-type zebrafish were injected at the one-cell stage. Microinjections were carried out five times to generate approximately 300 surviving embryos. Injected founder fish were mated to wild-type fish and their progeny.

Two lines of Tg:pomc-pttg transgenics were analyzed and showed similar metabolic features. All depicted data are derived from F2 and F3 transgenic progeny.

Maintenance of zebrafish and in vivo drug treatments Zebrafish embryos were maintained and raised as described²³. Dexamethasone (Sigma) was dissolved in distilled water, R-roscovitin (Selleck Chemicals), flavopiridol (Selleck Chemicals), PD0332991 (Selleck Chemicals), olomoucine (Cayman Chemical Co.), and CAY10578 (Cayman Chemical Co.) were dissolved in 0.2% DMSO at a stock concentration of 1 mM, and diluted in fish medium immediately before adding to live embryos at the stages indicated.

### Example 8

### RNA Whole-Mount in Situ Hybridization

A 1.0-kb zebrafish zPttg PCR product was subcloned into the pCR4-TOPO vector, which was subsequently linearized by NotI and transcribed with T3 polymerase to generate zPttg antisense mRNA. Pit-1 antisense mRNA was generated as described⁴⁶. Identification and isolation of zebrafish Tpit by Bioinformatic search of the Zebrafish Genome Database (http://www.ensembl.org/Danio_rerio; T. J. P. Hubbard et al., Ensembl 2007 Nucleic Acids Res. 2007 Vol. 35, Database issue: D610-D617), followed by DNA sequence analysis of isolated PCR products are described separately. In situ RNA hybridizations of whole-mount zebrafish embryos were performed as described⁴⁷.

### Example 9

### Histology and Immunohistochemistry

Adult zebrafish heads were fixed in 4% paraformaldehyde overnight and paraffin-embedded. H&E and immunohistochemical staining were performed on 5- µM sections. The streptavidin-biotin-peroxidase complex technique was used with rabbit anti-human ACTH antibodies (1:100; National Hormone and Peptide Program, National Institute of Diabetes and Digestive and Kidney Diseases; and A. F. Parlow, Harbor-University of California, Los Angeles, Medical Center, Los Angeles, CA). For immunofluorescence analysis, pituitaries were dissected and fixed. PCNA (1:100; Abcam) and ACTH (1:100) antibody staining were followed by corresponding secondary antibodies conjugated with Alexa 488 or Alexa 568 fluorescent dye (Abcam).

### Example 10

### Fluorescent Microcopy and Confocal Imaging

WT and transgenic embryos were examined at various developmental stages under a fluorescein isothiocyanate filter on an Axioplan-2 microscope (Carl Zeiss). Live embryo images were generated with an Axiocam video system (Carl Zeiss). Fluorescence intensity of POMC-GFP-positive cells was measured by the area of interest function in Openlab software (Improvision). For confocal imaging, images were captured in a Z-series by using a TCS SP confocal microscope (Leica Microsystems). GFP was detected at a spectral range from 507 to 550 nm, RFP from 585 to 690 nm, and DAPI from 460 to 480 nm. Images were prepared using Leica LCS lite, Volocity 5.2 (Improvision), and Photoshop 7.0 (Adobe). Pituitary GFP and RFP intensity and area were measured using Volocity 5.2 (Improvision). Lipid Staining. Liver cryosectionswere stained with oil redO(Sigma-Aldrich) per manufacturer's protocol. Lipid staining was scored based upon area distribution (>50% positive cells, 2 points; <50% but>10%, 1 point;<10%, 0 points) and intensity [strong, large (size of cell) lipid droplets, 2 points; weak, small (smaller than cell size) lipid droplets, 1 point; negative, 0 points].

### Example 11

### Blood Glucose Levels and Insulin Tolerance Tests

For blood glucose measurement, adult zebrafish were anesthetized in 0.04% tricaine methanesulfonate before tail section, and 2 µL tail blood applied to a glucometer test strip (OneTouch Ultra). For insulin tolerance test, adult zebrafish were given peritoneal insulin injection at a concentration of 0.1 U/100 mg body weight, followed by blood glucose measurement at different time points.

### Example 12

### Cortisol Radioimmunoassay

Fish were snap-frozen in liquid nitrogen, stored at -80 °C, homogenized on ice with a micro grinder (Eppendorf), then extracted with 500 µL of cold ethanol. After centrifugation for 10 min at 1,000 × g at 4 °C, the supernatant was recovered and evaporated and the resultant pellet resuspended in 25 µL of zero calibrator buffer for cortisol radioimmunoassay (Siemens). Radioactivity was counted and results calculated by COBRA II Auto Gamma (Perkin-Elmer).

### Example 13

### Mice

Animal experiments were performed in accordance with Cedars-Sinai Institutional Animal Care and Use committee guidelines. Mouse corticotroph tumor AtT20 cells (∼1 × 10⁵) were inoculated s.c. into 6-wk-old female nu/nu mice. Three days after tumor cell inoculation, animals were randomized to receive R-roscovitine 150 mg/kg twice daily or vehicle via oral gavage for 5 d each week. After 3 wk of treatment, mice were killed by CO₂ inhalation, and tumors were dissected, weighted, and snapfrozen for further analysis.

### Example 14

### Cell Culture and Transfection

Mouse corticotroph tumorAtT20 cells were cultured in DMEM supplemented with 10% FBS at 37 °C in 5% CO₂ for 24 h followed by treatment of R-roscovitine or vehicle (0.2%DMSO). Cells were replenished daily with R-roscovitine and maintained in medium for up to 48 h. siRNA transfections were performed in 70% to 80% confluent cells using Lipofectamine 2000 (Invitrogen) according to the manufacturer's protocol.

### Example 15

### Western Blot Analysis

Protein samples were prepared in RIPA buffer (Sigma), separated on NuPAGE Novex Bis-Tris Gels (Invitrogen), and transferred onto PVDF membrane (Millipore) before blotting with primary antibody [cyclin E, 1:500 (Abeam); PTTG, 1:1,000 (Abcam); β-actin, 1:5,000 (Sigma); and p21, 1:1,000, p27, 1:500, p57, 1:1,000, pRb821, 1:1,000 (Santa Cruz Biotechnology)] at 4 °C overnight. After washes with 0.5% Tween-20 in Tris-buffered saline solution, membranes were incubated with horseradish peroxidaselinked secondary antibody (GE Healthcare) and developed using ECL Western blotting detection reagents (GE Healthcare).

### Example 16

### Statistical Analysis

P value of total body cortisol levels were calculated by unpaired Student t test. Group differences in glucose levels were assessed by ANOVA.

### References

1. Nieman LK, Biller BM, Findling JW, et al. The diagnosis of Cushing's syndrome: an Endocrine Society Clinical Practice Guideline. J Clin Endocrinol Metab. May 2008;93(5):1526-1540.
2. Biller BM, Grossman AB, Stewart PM, et al. Treatment of adrenocorticotropin-dependent Cushing's syndrome: a consensus statement. J Clin Endocrinol Metab. Jul 2008;93(7):2454-2462.
3. Assie G, Bahurel H, Coste J, et al. Corticotroph tumor progression after adrenalectomy in Cushing's Disease: A reappraisal of Nelson's Syndrome. J Clin Endocrinol Metab. Jan 2007;92(1):172-179.
4. Boscaro M, Ludlam WH, Atkinson B, et al. Treatment of pituitary-dependent Cushing's disease with the multireceptor ligand somatostatin analog pasireotide (SOM230): a multicenter, phase II trial. J Clin Endocrinol Metab. Jan 2009;94(1):115-122.
5. Melmed S. Mechanisms for pituitary tumorigenesis: the plastic pituitary. J Clin Invest. Dec 2003;112(11):1603-1618.
6. Moons DS, Jirawatnotai S, Parlow AF, Gibori G, Kineman RD, Kiyokawa H. Pituitary hypoplasia and lactotroph dysfunction in mice deficient for cyclin-dependent kinase-4. Endocrinology. Aug 2002;143(8):3001-3008.
7. Melmed S. Acromegaly pathogenesis and treatment. J Clin Invest. Nov 2009;119(11):3189-3202.
8. Sotillo R, Renner O, Dubus P, et al. Cooperation between Cdk4 and p27kip1 in tumor development: a preclinical model to evaluate cell cycle inhibitors with therapeutic activity. Cancer Res. May 1 2005;65(9):3846-3852.
9. Roussel-Gervais A, Bilodeau S, Vallette S, et al. Cooperation between Cyclin E and p27Kip1 in Pituitary Tumorigenesis. Mol Endocrinol. Jul 21 2010.
10. Shapiro GI. Cyclin-dependent kinase pathways as targets for cancer treatment. J Clin Oncol. Apr 10 2006;24(11):1770-1783.
11. Malumbres M, Barbacid M. Cell cycle, CDKs and cancer: a changing paradigm. Nat Rev Cancer. Mar 2009;9(3):153-166.
12. Fabian MA, Biggs WH, 3rd, Treiber DK, et al. A small molecule-kinase interaction map for clinical kinase inhibitors. Nat Biotechnol. Mar 2005;23(3):329-336.
13. Vlotides G, Eigler T, Melmed S. Pituitary tumor-transforming gene: physiology and implications for tumorigenesis. Endocr Rev. Apr 2007;28(2):165-186.
14. Pei L, Melmed S. Isolation and characterization of a pituitary tumor-transforming gene (PTTG). Mol Endocrinol. Apr 1997;11(4):433-441.
15. Marangos P, Carroll J. Securin regulates entry into M-phase by modulating the stability of cyclin B. Nat Cell Biol. Apr 2008;10(4):445-451.
16. Romero F, Multon MC, Ramos-Morales F, et al. Human securin, hPTTG, is associated with Ku heterodimer, the regulatory subunit of the DNA-dependent protein kinase. Nucleic Acids Res. Mar 15 2001;29(6):1300-1307.
17. Tong Y, Tan Y, Zhou C, Melmed S. Pituitary tumor transforming gene interacts with Sp1 to modulate G1/S cell phase transition. Oncogene. Aug 16 2007;26(38):5596-5605.
18. Zou H, McGarry TJ, Bernal T, Kirschner MW. Identification of a vertebrate sister chromatid separation inhibitor involved in transformation and tumorigenesis. Science. Jul 16 1999;285(5426):418-422.
19. Bernal JA, Roche M, Mendez-Vidal C, Espina A, Tortolero M, Pintor-Toro JA. Proliferative potential after DNA damage and non-homologous end joining are affected by loss of securin. Cell Death Differ. Jan 2008;15(1):202-212.
20. Kim D, Pemberton H, Stratford AL, et al. Pituitary tumour transforming gene (PTTG) induces genetic instability in thyroid cells. Oncogene. Jul 14 2005;24(30):4861-4866.
21. Chesnokova V, Zonis S, Kovacs K, et al. p21(Cip1) restrains pituitary tumor growth. Proc Natl Acad Sci USA. Nov 11 2008;105(45):17498-17503.
22. Donangelo I, Gutman S, Horvath E, et al. Pituitary tumor transforming gene overexpression facilitates pituitary tumor development. Endocrinology. Oct 2006;147(10):4781-4791.
23. Liu NA, Huang H, Yang Z, et al. Pituitary corticotroph ontogeny and regulation in transgenic zebrafish. Mol Endocrinol. May 2003;17(5):959-966.
24. Camper SA, Saunders TL, Katz RW, Reeves RH. The Pit-1 transcription factor gene is a candidate for the murine Snell dwarf mutation. Genomics. Nov 1990;8(3):586-590.
25. Lamolet B, Pulichino AM, Lamonerie T, et al. A pituitary cell-restricted T box factor, Tpit, activates POMC transcription in cooperation with Pitx homeoproteins. Cell. Mar 23 2001;104(6):849-859.
26. Liu J, Lin C, Gleiberman A, et al. Tbx19, a tissue-selective regulator of POMC gene expression. Proc Natl Acad Sci USA. Jul 17 2001;98(15):8674-8679.
27. Bilodeau S, Vallette-Kasic S, Gauthier Y, et al. Role of Brg1 and HDAC2 in GR transrepression of the pituitary POMC gene and misexpression in Cushing disease. Genes Dev. Oct 15 2006;20(20):2871-2886.
28. Hsu HJ, Lin G, Chung BC. Parallel early development of zebrafish interrenal glands and pronephros: differential control by wt1 and fflb. Development. May 2003;130(10):2107-2116.
29. Alexander EL, Dooley KC, Pohajdak B, Xu BY, Wright JR, Jr. Things we have learned from tilapia islet xenotransplantation. Gen Comp Endocrinol. Sep 1 2006;148(2):125-131.
30. Muiesan ML, Lupia M, Salvetti M, et al. Left ventricular structural and functional characteristics in Cushing's syndrome. J Am Coll Cardiol. Jun 18 2003;41(12):2275-2279.
31. Iida H, Towatari M, Tanimoto M, Morishita Y, Kodera Y, Saito H. Overexpression of cyclin E in acute myelogenous leukemia. Blood. Nov 1 1997;90(9):3707-3713.
32. Jordan S, Lidhar K, Korbonits M, Lowe DG, Grossman AB. Cyclin D and cyclin E expression in normal and adenomatous pituitary. Eur J Endocrinol. Jul 2000;143(1):R1-6.
33. De Azevedo WF, Leclerc S, Meijer L, Havlicek L, Strnad M, Kim SH. Inhibition of cyclin-dependent kinases by purine analogues: crystal structure of human cdk2 complexed with roscovitine. Eur J Biochem. Jan 15 1997;243(1-2):518-526.
34. Montagnoli A, Valsasina B, Croci V, et al. A Cdc7 kinase inhibitor restricts initiation of DNA replication and has antitumor activity. Nat Chem Biol. Jun 2008;4(6):357-365.
35. Liu NA, Liu Q, Wawrowsky K, Yang Z, Lin S, Melmed S. Prolactin receptor signaling mediates the osmotic response of embryonic zebrafish lactotrophs. Mol Endocrinol. Apr 2006;20(4):871-880.
36. Bilodeau S, Roussel-Gervais A, Drouin J. Distinct developmental roles of cell cycle inhibitors p57Kip2 and p27Kip1 distinguish pituitary progenitor cell cycle exit from cell cycle reentry of differentiated cells. Mol Cell Biol. Apr 2009;29(7):1895-1908.
37. Woo YS, Isidori AM, Wat WZ, et al. Clinical and biochemical characteristics of adrenocorticotropin-secreting macroadenomas. J Clin Endocrinol Metab. Aug 2005;90(8):4963-4969.
38. Invitti C, Pecori Giraldi F, de Martin M, Cavagnini F. Diagnosis and management of Cushing's syndrome: results of an Italian multicentre study. Study Group of the Italian Society of Endocrinology on the Pathophysiology of the Hypothalamic-Pituitary- Adrenal Axis. J Clin Endocrinol Metab. Feb 1999;84(2):440-448.
39. Fukuoka H, Ben-Shlomo A, Mamelak A, et al. Inhibition of EGF Receptor (EGFR) Signaling in Cushing's Disease: A Novel Pathway for Abrogating STAT3 Regulation of ACTH. Endocrine Society Annual Meeting. San Diego, California 2010.
40. Fukuoka H, Vlotides G, Cooper O, Mizutani J, Ren S, Melmed S. Her2/Neu Receptor Signaling in Rat and Human Prolactinoma Cells: Novel Strategy for Targeted Prolactinoma Therapy. Endocrine Society Annual Meeting. San Diego, California 2010.
41. De Martino I, Visone R, Wierinckx A, et al. HMGA proteins up-regulate CCNB2 gene in mouse and human pituitary adenomas. Cancer Res. Mar 1 2009;69(5):1844-1850.
42. Quereda V, Malumbres M. Cell cycle control of pituitary development and disease. J Mol Endocrinol. Feb 2009;42(2):75-86.
43. Whittaker SR, Walton MI, Garrett MD, Workman P. The Cyclin-dependent kinase inhibitor CYC202 (R-roscovitine) inhibits retinoblastoma protein phosphorylation, causes loss of Cyclin D1, and activates the mitogen-activated protein kinase pathway. Cancer Res. Jan 1 2004;64(1):262-272.
44. McClue SJ, Blake D, Clarke R, et al. In vitro and in vivo antitumor properties of the cyclin dependent kinase inhibitor CYC202 (R-roscovitine). Int J Cancer. Dec 10 2002;102(5):463-468.
45. Legraverend M, Grierson DS. The purines: potent and versatile small molecule inhibitors and modulators of key biological targets. Bioorg Med Chem. Jun 15 2006;14(12):3987- 4006.
46. Bilodeau S, Roussel-Gervais A, Drouin J (2009) Distinct developmental roles of cell cycle inhibitors p57Kip2 and p27Kip1 distinguish pituitary progenitor cell cycle exit from cell cycle reentry of differentiated cells. Mol Cell Biol 29:1895-1908.
47. Camper SA, Saunders TL, Katz RW, Reeves RH (1990) The Pit-1 transcription factor gene is a candidate for the murine Snell dwarf mutation. Genomics 8:586-590.

Various embodiments of the invention are described above in the Detailed Description. While these descriptions directly describe the above embodiments, it is understood that those skilled in the art may conceive modifications and/or variations to the specific embodiments shown and described herein. Any such modifications or variations that fall within the purview of this description are intended to be included therein as well. Unless specifically noted, it is the intention of the inventors that the words and phrases in the specification and claims be given the ordinary and accustomed meanings to those of ordinary skill in the applicable art(s).

The foregoing description of various embodiments of the invention known to the applicant at this time of filing the application has been presented and is intended for the purposes of illustration and description. The present description is not intended to be exhaustive nor limit the invention to the precise form disclosed and many modifications and variations are possible in the light of the above teachings. The embodiments described serve to explain the principles of the invention and its practical application and to enable others skilled in the art to utilize the invention in various embodiments and with various modifications as are suited to the particular use contemplated. Therefore, it is intended that the invention not be limited to the particular embodiments disclosed for carrying out the invention.

It will be understood by those within the art that, in general, terms used herein are generally intended as "open" terms (*e.g.,* the term "including" should be interpreted as "including but not limited to," the term "having" should be interpreted as "having at least," the term "includes" should be interpreted as "includes but is not limited to," etc.).

## Claims

1. A composition comprising oleomoucine or R-roscovitine or salts thereof for use as a medicament for administration, in a therapeutically effective amount, to a mammalian subject in need of treating a pituitary corticotroph tumor, suppressing ACTH and/or corticosterone levels in a ACTH-secreting pituitary adenoma, inhibiting the growth of an ACTH-secreting pituitary adenoma, or treating Cushing's disease to treat the pituitary corticotroph tumor, suppress the ACTH and/or corticosterone levels in a ACTH-secreting pituitary adenoma, inhibit the growth of an ACTH-secreting pituitary adenoma, or treat Cushing's disease.

2. The composition for use according to claim 1, wherein the mammalian subject is in need of treating a pituitary corticotroph tumor and the pituitary corticotroph tumor is treated.

3. The composition for use according to claim 1, wherein the mammalian subject is in need of suppressing ACTH and/or corticosterone levels in an ACTH-secreting pituitary adenoma and the ACTH and/or corticosterone levels in the ACTH-secreting pituitary adenoma are suppressed.

4. The composition for use according to claim 1, wherein the mammalian subject is in need of inhibiting the growth of an ACTH-secreting pituitary adenoma and the growth of an ACTH-secreting pituitary adenoma is inhibited.

5. The composition for use according to claim 1, wherein the mammalian subject is in need of treating Cushing's disease and Cushing's disease is treated.

6. The composition for use according to claim 1, wherein composition comprises R-roscovitine or salts thereof.

## Patentansprüche

1. Zusammensetzung, umfassend Oleomoucin oder R-Roscovitin oder Salze davon zur Verwendung als ein Arzneimittel zur Verabreichung in einer therapeutisch wirksamen Menge an ein Säugetiersubjekt, das eine Behandlung eines pituitären corticotropen Tumors, eine Unterdrückung von ACTH- und/oder Corticosteronwerten in einem ACTH-sekretierenden pituitären Adenom, eine Hemmung des Wachstums eines ACTH-sekretierenden pituitären Adenoms oder eine Behandlung des Cushing-Syndroms zur Behandlung des pituitären corticotropen Tumors, eine Unterdrückung von ACTH- und/oder Corticosteronwerten in einem ACTH-sekretierenden pituitären Adenom, eine Hemmung des Wachstums eines ACTH-sekretierenden pituitären Adenoms oder eine Behandlung des Cushing-Syndroms benötigt.

2. Zusammensetzung zur Verwendung nach Anspruch 1, wobei das Säugetiersubjekt eine Behandlung eines pituitären corticotropen Tumors benötigt, und wobei der pituitäre corticotrope Tumor behandelt wird.

3. Zusammensetzung zur Verwendung nach Anspruch 1, wobei das Säugetiersubjekt eine Unterdrückung von ACTH- und/oder Corticosteronwerten in einem ACTH-sekretierenden pituitären Adenom benötigt, und wobei die ACTH- und/oder Corticosteronwerte in einem ACTH-sekretierenden pituitären Adenom unterdrückt werden.

4. Zusammensetzung zur Verwendung nach Anspruch 1, wobei das Säugetiersubjekt eine Hemmung des Wachstums eines ACTH-sekretierenden pituitären Adenoms benötigt, und wobei das Wachstum eines ACTH-sekretierenden pituitären Adenoms gehemmt wird.

5. Zusammensetzung zur Verwendung nach Anspruch 1, wobei das Säugetiersubjekt eine Behandlung des Cushing-Syndroms benötigt, und wobei das Cushings-Syndrom behandelt wird.

6. Zusammensetzung zur Verwendung nach Anspruch 1, wobei die Zusammensetzung R-Roscovitin oder Salze davon umfasst.

## Revendications

1. Composition comprenant de l'oléomoucine ou de la R-roscovitine ou des sels de celles-ci destinée à une utilisation en tant qu'un médicament pour une administration, dans une quantité thérapeutiquement efficace, chez un sujet mammalien qui a besoin de traiter une tumeur corticotrophe pituitaire, de supprimer l'ACTH et/ou les niveaux de cortisone dans un adénome pituitaire sécrétant de l'ACTH, d'inhiber la croissance d'un adénome pituitaire sécrétant de l'ACTH, ou de traiter la maladie de Cushing pour traiter la tumeur corticotrophe pituitaire, de supprimer l'ACTH et/ou les niveaux de corticostérone dans un adénome pituitaire sécrétant de l'ACTH, d'inhiber la croissance d'un adénome pituitaire sécrétant de l'ACTH, ou de traiter la maladie de Cushing.

2. Composition selon la revendication 1, dans laquelle le sujet mammalien a besoin de traiter une tumeur corticotrophe pituitaire et la tumeur corticotrophe pituitaire est traitée.

3. Composition selon la revendication 1, dans laquelle le sujet mammalien a besoin de supprimer l'ACT et/ou les niveaux de cortisone dans un adénome pituitaire sécrétant de l'ACTH et l'ACT et/ou les niveaux de cortisone dans un adénome pituitaire sécrétant de l'ACTH sont supprimés.

4. Composition selon la revendication 1, dans laquelle le sujet mammalien a besoin d'inhiber la croissance d'un adénome pituitaire sécrétant de l'ACTH et la croissance d'un adénome pituitaire sécrétant de l'ACTH est inhibée.

5. Composition selon la revendication 1, dans laquelle le sujet mammalien a besoin de traiter la maladie de Cushing et la maladie de Cushing est traitée.

6. Composition selon la revendication 1, dans laquelle la composition comprend de la R-roscovitine et des sels de celle-ci.


### SEQUENCE LISTING

<110> CEDARS-SINAI MEDICAL CENTER
   MELTED, Shlomo
   LIU, Ning-Ai
<120> TREATMENT OF PITUITARY CORTICOTROPH TUMORS USING R-ROSCOVITINE
<130> 67789-303WO0
<150> 61/409, 756 <151> 2010-11-03
<150> 61/483, 223 <151> 2011-05-06
<160> 27
<170> Patent In version 3.5
<210> 1
   <211> 23
   <212> DNA
   <213> Zebrafish
<400> 1
   aacgctggac cttagcgaag act 23
<210> 2
   <211> 32
   <212> DNA
   <213> Zebrafish
<400> 2
   tactagaaca ggtttcttta ttttcttgcg tg 32
<210> 3
   <211> 17
   <212> PRT
   <213> Artificial Sequence
<220>
   <223> synthetic construct
<400> 3
   Ala Cys Arg Arg Leu Phe Gly Pro Val Asp Ser Glu Gln Leu Ser Arg
   1               5                   10                  15
   Asp
<210> 4
   <211> 14
   <212> PRT
   <213> Artificial Sequence
<220>
   <223> synthetic construct
<400> 4
   Ala Trp Glu Arg Val Arg Gly Leu Gly Leu Pro Lys Leu Tyr
   1               5                   10
<210> 5
   <211> 20
   <212> PRT
   <213> Artificial Sequence
<220>
   <223> synthetic construct
<400> 5
   Lys Arg Arg Gln Thr Ser Met Thr Asp Phe Tyr His Ser Lys Arg Arg
   1               5                   10                  15
   Leu Ile Phe Ser
               20
<210> 6
   <211> 20
   <212> PRT
   <213> Artificial Sequence
<220>
   <223> synthetic construct
<400> 6
   Lys Arg Arg Gln Thr Ser Met Thr Asp Phe Tyr His Ser Lys Arg Arg
   1               5                   10                  15
   Leu Ile Phe Ser
               20
<210> 7
   <211> 20
   <212> PRT
   <213> Artificial Sequence
<220>
   <223> synthetic construct
<400> 7
   Lys Arg Arg Gln Thr Ser Ala Thr Asp Phe Tyr His Ser Lys Arg Arg
   1               5                   10                  15
   Leu Ile Phe Ser
               20
<210> 8
   <211> 26
   <212> PRT
   <213> Artificial Sequence
<220>
   <223> synthetic construct
<400> 8
   Gly Arg Lys Arg Arg Gln Thr Ser Met Thr Asp Phe Tyr His Ser Lys
   1               5                   10                  15
   Arg Arg Leu Ile Phe Ser Lys Arg Lys Pro
               20                  25
<210> 9
   <211> 20
   <212> PRT
   <213> Artificial Sequence
<220>
   <223> synthetic construct
<400> 9
   Lys Arg Arg Gln Thr Ser Met Thr Asp Phe Tyr His Ser Lys Arg Arg
   1               5                   10                  15
   Leu Ile Phe Ser
               20
<210> 10
   <211> 20
   <212> PRT
   <213> Artificial Sequence
<220>
   <223> synthetic construct
<400> 10
   Lys Arg Arg Ala Thr Ser Met Thr Asp Phe Tyr His Ser Lys Arg Arg
   1               5                   10                  15
   Leu Ile Phe Ser
               20
<210> 11
   <211> 20
   <212> PRT
   <213> Artificial Sequence
<220>
   <223> synthetic construct
<400> 11
   Lys Arg Arg Gln Thr Ser Ala Thr Asp Phe Tyr His Ser Lys Arg Arg
   1               5                   10                  15
   Leu Ile Phe Ser
               20
<210> 12
   <211> 20
   <212> PRT
   <213> Artificial Sequence
<220>
   <223> synthetic construct
<400> 12
   Lys Arg Arg Gln Thr Ser Met Thr Asp Phe Tyr His Ser Lys Arg Arg
   1               5                   10                  15
   Leu Ile Ala Ser
               20
<210> 13
   <211> 26
   <212> PRT
   <213> Artificial Sequence
<220>
   <223> synthetic construct
<400> 13
   Gly Arg Lys Arg Arg Gln Thr Ser Met Thr Asp Phe Tyr His Ser Lys
   1               5                   10                  15
   Arg Arg Leu Ile Phe Ser Lys Arg Lys Pro
               20                  25
<210> 14
   <211> 26
   <212> PRT
   <213> Artificial Sequence
<220>
   <223> synthetic construct
<400> 14
   Gly Arg Lys Arg Arg Gln Thr Ser Met Thr Asp Phe Tyr His Ser Lys
   1               5                   10                  15
   Arg Arg Leu Ile Phe Ser Lys Arg Lys Pro
               20                  25
<210> 15
   <211> 26
   <212> PRT
   <213> Artificial Sequence
<220>
   <223> synthetic construct
<400> 15
   Gly Arg Lys Arg Arg Gln Thr Ser Met Thr Asp Phe Tyr His Ser Lys
   1               5                   10                  15
   Arg Arg Leu Ile Phe Ser Lys Arg Lys Pro
               20                  25
<210> 16
   <211> 8
   <212> PRT
   <213> Artificial Sequence
<220>
   <223> synthetic construct
<400> 16
   His Ala Lys Arg Arg Leu Ile Phe
   1               5
<210> 17
   <211> 19
   <212> PRT
   <213> Artificial Sequence
<220>
   <223> synthetic construct
<400> 17
   Asp Ala Ala Arg Glu Gly Phe Leu Asp Thr Leu Val Val His Arg Ala
   1               5                   10                  15
   Gly Ala Arg
<210> 18
   <211> 8
   <212> PRT
   <213> Artificial Sequence
<220>
   <223> synthetic construct
<400> 18
   Pro Val Lys Arg Arg Leu Asp Leu
   1               5
<210> 19
   <211> 14
   <212> PRT
   <213> Artificial Sequence
<220>
   <223> synthetic construct
<400> 19
   Ser Asn Pro Pro Lys Pro Leu Lys Lys Arg Phe Asp Ile Glu
   1 5 10
<210> 20
   <211> 8
   <212> PRT
   <213> Artificial
<220>
   <223> synthetic construct
<220>
   <221> MISC_FEATURE
   <222> (3)..(3)
   <223> Xaa can be aminobutyric acid (Abu)
<400> 20
   Ala Ala Xaa Arg Lys Leu Phe Gly
   1 5
<210> 21
   <211> 22
   <212> PRT
   <213> Artificial Sequence
<220>
   <223> synthetic construct
<400> 21
   Thr Tyr Thr Lys Lys Gln Val Leu Arg Met Glu His Leu Val Leu Lys
   1 5 10 15
   Val Leu Thr Phe Asp Leu
   20
<210> 22
   <211> 22
   <212> PRT
   <213> Artificial Sequence
<220>
   <223> synthetic construct
<400> 22
   Thr Tyr Thr Lys Lys Gln Val Leu Arg Met Glu His Leu Val Leu Lys
   1 5 10 15
   Val Leu Thr Phe Asp Leu
   20
<210> 23
   <211> 9
   <212> PRT
   <213> Artificial Sequence
<220>
   <223> synthetic construct
<400> 23
   Asn Asx Ile Arg Trp Ile Met Tyr Phe
   1               5
<210> 24
   <211> 202
   <212> PRT
   <213> Homo sapiens
<400> 24
   Met Ala Thr Leu Ile Tyr Val Asp Lys Glu Asn Gly Glu Pro Gly Thr
   1               5                   10                  15
   Arg Val Val Ala Lys Asp Gly Leu Lys Leu Gly Ser Gly Pro Ser Ile
               20                  25                  30
   Lys Ala Leu Asp Gly Arg Ser Gln Val Ser Thr Pro Arg Phe Gly Lys
           35                  40                  45
   Thr Phe Asp Ala Pro Pro Ala Leu Pro Lys Ala Thr Arg Lys Ala Leu
       50                  55                  60
   Gly Thr Val Asn Arg Ala Thr Glu Lys Ser Val Lys Thr Lys Gly Pro
   65                  70                  75                  80
   Leu Lys Gln Lys Gln Pro Ser Phe Ser Ala Lys Lys Met Thr Glu Lys
                   85                  90                  95
   Thr Val Lys Ala Lys Ser Ser Val Pro Ala Ser Asp Asp Ala Tyr Pro
               100                 105                 110
   Glu Ile Glu Lys Phe Phe Pro Phe Asn Pro Leu Asp Phe Glu Ser Phe
           115                 120                 125
   Asp Leu Pro Glu Glu His Gln Ile Ala His Leu Pro Leu Ser Gly Val
       130                 135                 140
   Pro Leu Met Ile Leu Asp Glu Glu Arg Glu Leu Glu Lys Leu Phe Gln
   145                 150                 155                 160
   Leu Gly Pro Pro Ser Pro Val Lys Met Pro Ser Pro Pro Trp Glu Ser
                   165                 170                 175
   Asn Leu Leu Gln Ser Pro Ser Ser Ile Leu Ser Thr Leu Asp Val Glu
               180                 185                 190
   Leu Pro Pro Val Cys Cys Asp Ile Asp Ile
           195                 200
<210> 25
   <211> 199
   <212> PRT
   <213> Mouse
<400> 25
   Met Ala Thr Leu Ile Phe Val Asp Lys Asp Asn Glu Glu Pro Gly Arg
   1               5                   10                  15
   Arg Leu Ala Ser Lys Asp Gly Leu Lys Leu Gly Thr Gly Val Lys Ala
               20                  25                  30
   Leu Asp Gly Lys Leu Gln Val Ser Thr Pro Arg Val Gly Lys Val Phe
           35                  40                  45
   Asn Ala Pro Ala Val Pro Lys Ala Ser Arg Lys Ala Leu Gly Thr Val
       50                  55                  60
   Asn Arg Val Ala Glu Lys Pro Met Lys Thr Gly Lys Pro Leu Gln Pro
   65                  70                  75                  80
   Lys Gln Pro Thr Leu Thr Gly Lys Lys Ile Thr Glu Lys Ser Thr Lys
                   85                  90                  95
   Thr Gln Ser Ser Val Pro Ala Pro Asp Asp Ala Tyr Pro Glu Ile Glu
               100                 105                 110
   Lys Phe Phe Pro Phe Asn Pro Leu Asp Phe Glu Ser Phe Asp Leu Pro
           115                 120                 125
   Glu Glu His Gln Ile Ser Leu Leu Pro Leu Asn Gly Val Pro Leu Met
       130                 135                 140
   Thr Leu Asn Glu Glu Arg Gly Leu Glu Lys Leu Leu His Leu Gly Pro
   145                 150                 155                 160
   Pro Ser Pro Leu Lys Thr Pro Phe Leu Ser Trp Glu Ser Asp Pro Leu
                   165                 170                 175
   Tyr Ser Pro Pro Ser Ala Leu Ser Thr Leu Asp Val Glu Leu Pro Pro
               180                 185                 190
   Val Cys Tyr Asp Ala Asp Ile
           195
<210> 26
   <211> 188
   <212> PRT
   <213> Xenopus
<400> 26
   Met Ala Thr Val Val Phe Val Asp Gln Glu Asn Gly Asp Val Gly Ser
   1               5                   10                  15
   Ala Leu His Lys Asp Arg Gly Met Phe Leu Ser Ser Lys Thr Gln Ser
               20                  25                  30
   Arg Lys Ala Val Ala Ser Leu Pro Gly Lys Val Phe Gly Lys Ser Glu
           35                  40                  45
   Met Val Ser Lys Pro Ser Arg Lys Ala Leu Gly Asn Val Asn Lys Gln
       50                  55                  60
   Ile Leu Pro Lys Thr Ala Ala Thr Ala Gln Lys Ser Asp Leu Lys Gln
   65                  70                  75                  80
   Lys Ser Thr Val Pro Ile Gly Lys Lys Val Cys Ser Ser Lys Gln Pro
                   85                  90                  95
   Val Lys Asp Leu Tyr Pro Glu Ile Glu His Phe Val Pro Tyr Asn Pro
               100                 105                 110
   Leu Asp Phe Glu Ser Phe Asp Val Pro Glu Asp His Lys Leu Ser His
           115                 120                 125
   Leu Cys Leu Ala Gly Val Ser Leu Leu Val His Glu Asn Glu Val Ala
       130                 135                 140
   Arg Phe Asn Ala Leu Thr Asp Ile Gln Leu Cys Pro Leu Glu Met Pro
   145                 150                 155                 160
   Ser Leu Asn Met Val Ser Asp Tyr Leu Pro Phe Ile Ala Ala Leu Asp
                   165                 170                 175
   Asp Ile Thr Val Asp Leu Pro Pro Val Glu Asp Tyr
               180                 185
<210> 27
   <211> 182
   <212> PRT
   <213> Zebrafish
<400> 27
   Met Glu Thr Met Ile Tyr Met Asp Gln Glu Asn Gly Arg Leu Met Thr
   1               5                   10                  15
   Pro Ala Ile Lys Ser Arg Gln Asn Arg Leu His Ser Ala Pro Asp Gln
               20                  25                  30
   Cys Leu Arg Thr Pro Leu Asn Gly Lys Ala His Leu Gly Ala Pro Leu
           35                  40                  45
   Gln Ser Ser Arg Lys Ala Leu Gly Val Ile Asn Lys Ile Val Asn Gln
       50                  55                  60
   Thr Ser Ser His Glu Ala Glu Glu Lys Thr Lys Pro Ala Glu Ala Lys
   65                  70                  75                  80
   Cys Lys Val Pro Thr Pro Leu Pro Thr Glu Glu Leu Pro Glu Ile Glu
                   85                  90                  95
   Lys Phe Phe Pro Tyr Asn Pro Ser Glu Phe Glu Cys Tyr Ser Val Pro
               100                 105                 110
   Asp Glu Val Tyr Leu Ser Arg Phe Ser Leu Ala Gly Leu Gly Lys Gln
           115                 120                 125
   Thr Trp Leu Pro Ala Ser Pro Val Glu Glu Phe Ile Glu Pro Cys Leu
       130                 135                 140
   Pro Leu Ser Pro Leu Lys Met Pro Lys Glu Val Glu Tyr Gly Asp Glu
   145                 150                 155                 160
   Val Glu Ala Phe Leu Gln Thr Ile Asn Glu Leu Thr Glu Val Asp Leu
                   165                 170                 175
   Pro Pro Glu Cys Asp Phe
               180